(19) Europäisches Patentamt / European Patent Office / Office européen des brevets

(11) **EP 2 961 848 B1**

(12) **EUROPEAN PATENT SPECIFICATION**

(45) Date of publication and mention of the grant of the patent:
**11.04.2018 Bulletin 2018/15**

(51) Int Cl.:
*C12Q 1/48* (2006.01)    *G01N 33/92* (2006.01)

(21) Application number: **14707148.4**

(86) International application number:
**PCT/EP2014/053844**

(22) Date of filing: **27.02.2014**

(87) International publication number:
**WO 2014/131836 (04.09.2014 Gazette 2014/36)**

(54) **ANALYSIS OF THE ESTERIFICATION LEVEL OF CEREBROSTEROL AS A TOOL FOR DIAGNOSIS AND/OR PROGNOSIS OF NEURODEGENERATION**

ANALYSE DES VERESTERUNGSGRADES VON CEREBROSTEROL ALS MITTEL ZUR DIAGNOSE UND/ODER PROGNOSE VON NEURODEGENERATION

ANALYSE DU DEGRÉ D'ESTÉRIFICATION DU CÉRÉBROSTÉROL COMME OUTIL DE DIAGNOSTIC ET/OU DE PRONOSTIC D'UNE NEURODÉGÉNÉRESCENCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2013 IT RM20130112**

(43) Date of publication of application:
**06.01.2016 Bulletin 2016/01**

(73) Proprietor: **Trase S.r.l.**
**80132 Napoli (NA) (IT)**

(72) Inventor: **Abrescia, Paolo**
**80122 Napoli (IT)**

(74) Representative: **Capasso, Olga et al**
**De Simone & Partners SpA**
**Via Vincenzo Bellini, 20**
**00198 Roma (IT)**

(56) References cited:
**WO-A1-2012/064501   US-A1- 2009 029 473**
**US-A1- 2009 286 272   US-A1- 2011 207 230**

- **GILL S ET AL: "Sterol regulators of cholesterol homeostasis and beyond: The oxysterol hypothesis revisited and revised", PROGRESS IN LIPID RESEARCH, PERGAMON PRESS, PARIS, FR, vol. 47, no. 6, 1 November 2008 (2008-11-01), pages 391-404, XP025472190, ISSN: 0163-7827, DOI: 10.1016/J.PLIPRES.2008.04.002 [retrieved on 2008-05-06]**

**Description**

**TECHNICAL FIELD**

The present invention relates to a method for the diagnosis and/or prognosis or for the monitoring of the activity of the enzyme LCAT in the central nervous system, said method comprising the steps of measuring the amount of non-esterified cerebrosterol ($[24(S)OH-C_{NE}]$) and the amount of total cerebrosterol ($[24(S)OH-C_{TOT}]$) in a biological fluid and calculating the amount of esterified cerebrosterol ($[24(S)OH-CE]$), in which $[24(S)OH-CE] = [24(S)OH-C_{TOT}] - [24(S)OH-C_{NE}]$. In addition, the determination of $[24(S)OH-C_{NE}]$ and $[24(S)OH-CE]$ enables the diagnosis and/or prognosis or monitoring of the efficacy of a therapeutic treatment and/or the screening of a therapeutic treatment of a neurodegenerative disease.

**PRIOR ART**

Diseases of the central nervous system (CNS) constitute one of the largest segments and one of the most rapidly growing segments in the pharmaceutical market due to the unsatisfied demand for diagnosis and therapy. Neurodegenerative diseases represent approximately half of this segment and have the greatest tendency for growth; in particular, the growth potential for the primary diseases, that is to say Alzheimer's disease (AD) Parkinson's disease (PD), multiple Sclerosis (MS), Huntington's disease (HD) and amyotrophic lateral sclerosis (ALS), is of the exponential type in so far as it reflects the rise in life expectancy in developed countries, the cost of the therapies and above all the demand for more effective therapies. Many neurodegenerative diseases, including those specified above, cannot be diagnosed until they present severe symptoms. It is widely believed in research and in clinical practice that early diagnosis is the only tool for therapeutic interventions that can prevent the exacerbation of the symptoms and progression of the pathology and, in addition, for testing new drugs and new technologies. In particular, the assessment of the initial phases of the pathology is the most powerful tool for developing new drugs and for validating therapeutic treatments because the focus of the therapy is shifted from late and acute interventions to early, minimally invasive interventions. There is currently no cure enabling remission from neurodegenerative diseases. The therapies used today have little effect on the improvement of the symptoms, pose serious safety problems due to a multitude of side effects (including severe side effects), and, above all do not significantly influence the progression of the neurodegeneration.

Each type of neurodegenerative disease, although characterised by particular symptoms and specific changes in terms of both cellular function and biochemical processes, has features common to other neurodegenerative diseases, such as inflammation, oxidative stress and hypercholesterolemia [1-10]. Epidemiological, biochemical and pharmacological studies have provided growing evidence that there are correlations between these phenomena; in particular, both the condition of AD and that of mild cognitive impairment (MCI), which generally precedes AD, is accompanied by hypercholesterolemia [11-23]. Cholesterol, released by the CNS even in pathologies associated with demyelination, can accumulate in the brain as a cause or effect of neurodegeneration mechanisms which include oxidative stress [16, 23-28]. It is commonly believed that such an accumulation is preceded by various physiological processes of the CNS, some of which limit the synthesis of cholesterol whereas others promote the removal of the excess cholesterol, encouraging the transport of cholesterol into the bloodstream for elimination in the liver through the formation of bile acids [2, 6, 29-36]. A process that might promote the removal of cholesterol from the CNS is based on the enzymatic esterification of cholesterol, followed by the incorporation of the esters into lipoproteins crossing the blood-brain barrier [2, 6, 35-40]. Another process is based on the enzymatic conversion of cholesterol into its metabolite, cerebrosterol or 24(S)OH-C, which can freely diffuse through the blood-brain barrier [2, 6, 40-44]. However, the fact that the majority of this metabolite is in esterified form in the cerebrospinal fluid (CSF) [41, 45] has prompted the hypothesis that this metabolite is also transported essentially in the bloodstream as a lipoprotein component [41, 46, 47]. The removal of 24(S)OH-C (understood as a non-esterified form, referred to hereinafter as $24(S)OH-C_{NE}$) from the CNS appears to be necessary, similarly to the removal of cholesterol because neurotoxic effects have been suggested for both of these steroids when they accumulate at levels greater than physiological levels [16, 22, 26, 31, 48-56]. In particular, increased levels of total 24(S)OH-C (referred to hereinafter as $24(S)OH-C_{TOT}$ to indicate the total quantity of $24(S)OH-C_{NE}$ and forms esterified with fatty acids, referred to hereinafter as 24(S)OH-CE) in the CSF of patients suffering from AD are believed to reflect the accumulation of cholesterol and neurodegeneration [45, 57-59]. The enzyme capable to esterify with fatty acids whether cholesterol or $24(S)OH-C_{NE}$ is LCAT (lecithin-cholesterol acyltransferase, E.C. 2.3.1.43), which is normally present both in CSF [45, 60-62], in so far as this is secreted by the astrocytes [63-65], and in blood plasma [66, 67]. Proof that LCAT is capable of esterifying $24(S)OH-C_{NE}$ with chains of fatty acids containing a fluorescent tracer has been obtained recently [68]. This enzyme is stimulated by the apolipoproteins E (ApoE) and A-I (ApoA-I), which then massively relieve the load of steroid esters for forming lipoproteins, with an inner nucleus of concentrated esters, addressed to the bloodstream [66-69]. It is important to emphasise that, in physiological conditions, the apolipoproteins and some other factors (for example the peptide amyloid-β) could mediate the transport of sterols, in non-esterified form, from the CNS to the bloodstream [70-76], however the transported load would be limited to the direct and limited interaction between transporters and sterols and would therefore probably be sufficient for normal removal of the sterols that are continuously produced in the CNS in physiological conditions. Instead, in conditions in which there is an excess of sterols to be transported to the bloodstream, the assistance of LCAT could play a critical role, because the apolipoproteins can compact large quantities of steroid esters, embedded into lipoproteins addressed to the liver. Haptoglobin (Hpt), a protein secreted by several types of cells (including astrocytes and oligodendrocytes [77-81]), protects ApoE and ApoA-I from structural and functional damage caused by oxidative species [82, 83], which are normally produced by the nervous cells. Since the level of Hpt appears significantly reduced in the CFS of patients suffering from AD and MS [84, 85], the activity of antioxidant protection of this protein could be limited and unable to protect the apolipoproteins from losing their stimulating function LCAT [82]. In the same patients, the activity of LCAT in the CFS is almost halved as compared to normal levels [61]. LCAT is also a target of attack, degrading enzyme structure and function, by oxidative species, such as peroxynitrite [86, 87] and some products of lipid oxidation [88-90], which increase in conditions of oxidative stress such as those associated with inflammation, and cupric ions [91], commonly present in the CSF [92]. Therefore, oxidative stress damages both LCAT and its stimulators, with the likely result that both cholesterol and 24(S)OH-$C_{NE}$ could be poorly esterified and eliminated from the CNS. This hypothesis, which links oxidative stress to the accumulation of cholesterol and 24(S)OH-$C_{NE}$ in the CNS, is the subject of intense scientific research which, currently, has confirmed that oxidative stress and inflammation induce the expression of the enzyme that converts cholesterol to 24(S)OH-$C_{NE}$ [93-96]. The activity of LCAT in the CNS represents a biosensor of the oxidative stress in this compartment and it is reasonable to assume that it could be reduced by oxidative agents even in conditions of normal or reduced synthesis of cholesterol and 24(S)OH-$C_{NE}$, for example conditions associated with therapy with statins.

The diagnosis of neurodegenerative diseases is essentially based on biomarkers and/or image analysis techniques, particularly with respect to anatomical structures (MRI) [97, 98] or with respect to the glucose metabolism (FDG-PET), the formation of aggregates of amyloid-β (amyloid-β PET) or the cerebral blood flow (SPECT) [98, 99-107]. The diagnosis, particularly for AD, is often confirmed *post mortem* and, when confirmed during life, is useful only for monitoring the progression of the neurodegeneration when the symptoms are clear and the pathology cannot be stopped [108-112]. The most commonly used biomarkers of AD are the peptide amyloid-β and the phosphorylated protein tau, as described in a large number of scientific publications. However these mainly concern advanced phases of the disease. For early diagnosis of MS, new diagnostic systems are being prepared and are based on the levels of some biomarkers and image analysis data [113-117], however the diagnosis for this disease is currently obtained in advanced phases of the disease, taking into account magnetic resonance analyses, immunochemical analyses and the overall picture of disability indices for assessing the severity of the pathological state. Only some biomarkers for early diagnosis of PD, ALS and HD have been proposed, and, among these, a few appear to promise accuracy and specificity [118-121], but have not yet been validated. For many years, there has been a considerable commitment to find and test new biomarkers that make it possible to diagnose early these neurodegenerative diseases, because it is only possible with timely interventions in the pre-symptomatic phases or initial symptomatic phases to attempt to stem these diseases or at least slow down the progression thereof. Considering neurodegenerative diseases as a whole, a large number of molecular biomarkers, detectable in CSF and/or in plasma, have been proposed. In particular, more than 100 biomarkers of the CSF have been proposed for AD [122], of which the vast majority can serve to confirm advanced symptomatic phases of the disease; only a minority of these biomarkers could serve for early diagnosis (or diagnosis of the preclinical and presymptomatic phase) or for prediction of the onset of this disease (or the conversion from normal cognitive ability to mild cognitive impairment) [112, 123-136]. Such numbers clearly reflect the efforts of the research to meet the urgent need to make up for the lack of valid criteria and methods for early diagnosis or prognosis. A further problem is early differential diagnosis, that is to say the validation and use of biomarkers specific to one of the neurodegenerative diseases. It is commonly believed that, in addition to the possibility that differently altered levels of a biomarker can distinguish different pathologies, algorithms (which include the levels of other biomarkers or the level of a biomarker associated with genetic risk factors, image analysis data and/or clinical examination and cognitive test data) can be developed for differential diagnosis.

The genetic predisposition to AD based on the presence of the allele ε4 (or *apoE4*) of ApoE in the genome has been suggested both for the inefficient role that the gene expression product ApoE4 would have (compared with the other haplotypes ApoE2 and ApoE3, resulting respectively from the expression of the alleles ε2 and ε3) in the solubilisation of aggregates of amyloid-β and other physiological roles, and for the effect of ApoE4 to increase the production of toxic peptide fragments in the neurones [137, 138]. The genetic risk of *apoE4* has never been interpreted on the basis of the known inefficient stimulation of ApoE4 (compared with the other haplotypes ApoE2 and ApoE3) on the activity of LCAT. Although it is now commonly expected that hypercholesterolemia in the CNS is a factor of neurodegeneration (which proceeds the accumulation and depositing of amyloid-β, and hyperphosphorylation of tau in AD), the activity of LCAT has not yet been identified as one of the key systems for the removal of cholesterol and 24(S)OH-$C_{NE}$ from the CNS. Although it is now commonly accepted that oxidative stress in the CNS is a factor common to all neurodegenerative diseases, a specific target of the action of oxidative species in the CSF which is involved in cell survival mechanisms and is functionally insufficient with the onset and progression of neurodegeneration has not yet been identified. In addition, plasma biomarkers of the early phases of neurodegeneration that are involved in the removal of cholesterol and 24(S)OH-$C_{NE}$ or that, above all, reflect the oxidative stress in the CNS have not yet been discovered.

WO2012/064501 refers to derivatives of 24-S-hydroxycholesterol (indicated in the present invention as 24(S)OH-$C_{NE}$), e.g. 3-O-succinoyl-24-S-hydroxycholesterol, and to methods of evaluating progression of multiple sclerosis in a patient, the method comprising measuring 24-S-hydroxycholesterol (corresponding to 24(S)OH-$C_{TOT}$ as identified in the present invention) in blood plasma or serum. The International Application doesn't refer to forms of 24-S-hydroxycholesterol esterified with fatty acids (indicated in the present invention as 24(S)OH-CE).

US2009/286272 e.g. refers to the use of an oxysterol (24S-hydroxycholesterol (indicated in the present invention as deuterated 24(S)OH-$C_{NE}$)) as an internal standard in the determination of accumulation of oxysterols in methods of diagnosis and screening using biomarkers for diseases including neurodegenerative diseases. Although oxysterols are included in a class of lipid biomarkers, said application doesn't refer to esterified forms of 24-S-hydroxycholesterol (indicated in the present invention as 24(S)OH-CE) and, in particular, to the measurements of both 24(S)OH-$C_{NE}$ and 24(S)OH-$C_{TOT}$ levels for calculating the 24(S)OH-CE level and therefore evaluating the activity of the enzyme converting 24(S)OH-$C_{NE}$ into 24(S)OH-$C_{TOT}$.

Some oxysterols, such as 24(S)OH-$C_{TOT}$, have been studied for their possible use as biomarkers of neurodegeneration [36, 44, 45, 139-143]. As in US2009/286272, also in these studies, the level of these oxysterols was considered as the "total" of esterified forms and non-esterified forms. This approach does not take into account the fact that a major part of the 24(S)OH-$C_{TOT}$ is constituted by 24(S)OH-CE, resulting from the activity of LCAT, and has produced data that do not reflect neurodegenerative events relating to dysfunctions of this enzyme, caused by critical factors such as oxidative stress or changes in the synthesis and secretion of the enzyme. Many studies however suggest that the level of 24(S)OH-$C_{TOT}$ in the CNS (or in the CSF) may reflect neurodegenerative conditions, although in such studies there is a lack of support of longitudinal analyses and the *post-mortem* confirmation of such hypotheses and although in such studies it has been observed that the analysis of such a level in the plasma has limited [36] or no [45, 57] diagnostic power. The limited or lack of diagnostic power of the plasma level of 24(S)OH-$C_{TOT}$, encountered in the above-mentioned studies, weakens the expectations on the effective diagnostic power of the level of this oxysterol in the CSF. In fact, circulating 24(S)OH-$C_{TOT}$ is derived almost exclusively from the CNS, and its plasma level should therefore also unequivocally have diagnostic power similar to that of 24(S)OH-$C_{TOT}$ in the CSF. One study indicates that the plasma level of 24(S)OH-$C_{TOT}$ and of the quantitative ratio between this specific level and that of another oxysterol (27-hydroxycholesterol) increases in conditions of cerebrovascular disease [144], a pathological condition associated with early phases of AD. In this case too, the level of 24(S)OH-$C_{TOT}$ has been considered without taking into account the quantitative contribution of 24(S)OH-CE. Therefore, the role of biomarkers of early phases of neurodegeneration could be conferred at plasma level to 24(S)OH-$C_{TOT}$, but such a putative biomarker would reflect only the accumulation of cholesterol in the CNS and therefore would be insufficient for evaluating the neurodegenerative conditions of patients subjected to therapy with statins, which, as is well known, are widely used by the majority of the entire population of middle-aged and elderly people (that is to say by subjects at risk of neurodegeneration), and which, as widely indicated in the literature, have demonstrated efficacy in reducing the synthesis of cholesterol in the CNS. In addition, it is known that other types of drugs which lower the plasma level of cholesterol (such as, but not exclusively, niacin or inhibitors of the enzyme cholesteryl-ester acyltransferase), and hypercholesterolemia can significantly modify the level of 24(S)OH-$C_{TOT}$ in the bloodstream. Lastly, the use of the plasma level of 24(S)OH-$C_{TOT}$ as a biomarker of neurodegeneration does not provide any indication of the redox state in the CNS, the alteration of which precedes the synthesis of 24(S)OH-$C_{NE}$ [145].

## SUMMARY OF THE INVENTION

The present invention relates to the measurement of a biomarker specific for hypercholesterolemia and oxidative stress conditions associated with neurodegenerative phenomena. The biomarker is represented by the enzymatic activity of esterification of 24(S)OH-$C_{NE}$ in the interstitial fluid and cerebrospinal fluid of the CNS. The level of this biomarker, measured as the ratio between the amount of esterified (with fatty acids) and non-esterified forms of cerebrosterol (24(S)OH-CE and 24(S)OH-$C_{NE}$ respectively) or as the percentage or fraction of the amount of 24(S)OH-CE relative to the total amount of cerebrosterol (24(S)OH-$C_{TOT}$), can also be determined in the blood plasma. It must therefore be understood that the present invention does not relates to the level of 24(S)OH-$C_{TOT}$ but to the level of 24(S)OH-CE, which reflects a phenomenon different from that reflected by the level of 24(S)OH-$C_{TOT}$. Actually the level of 24(S)OH-$C_{TOT}$ reflects the activity of the enzyme CYP46A1 that synthesises 24(S)OH-$C_{NE}$, whereas the level of 24(S)OH-CE reflects the activity of the enzyme LCAT that esterifies 24(S)OH-$C_{NE}$. This enzyme is broken down by oxidative stress, which, together with the accumulation of cholesterol and 24(S)OH-$C_{NE}$, is neurotoxic. Therefore the analysis of the activity of the esterification of 24(S)OH-$C_{NE}$ is a new way of diagnosing early conditions of hypercholesterolemia and oxidative stress in the CNS, which are considered neurodegenerative risk factors. The present invention also relates to the diagnostic use of the above-mentioned new biomarker of neurodegeneration for evaluating the response of patients to treatments with drugs currently used for the therapy of neurodegenerative diseases. This invention also relates to the use of the above-mentioned new biomarker of neurodegeneration for evaluating the duration and intensity of treatment with drugs currently used for the therapy of neurodegenerative diseases. The present invention relates to the use of the above-mentioned biomarker of neurodegeneration for evaluating the efficacy of test drugs in new clinical treatments of neurodegenerative diseases. The use of the above-mentioned LCAT activity as a therapeutic target in the development of new drugs aimed at impeding the alteration of the level of the above-mentioned biomarker of neurodegeneration is an integral part of the present invention.

24(S)OH-$C_{NE}$ is a molecule synthesised by the enzymatic insertion of a hydroxyl group in the structure of the cholesterol, an event occurring almost exclusively in the CNS. In the present invention, proof is provided that the enzyme LCAT, known for the activity of esterification of cholesterol, also esterifies 24(S)OH-$C_{NE}$ and prevents the toxic effect of this specific oxysterol on neurons in culture. In addition, it has been found that conditions of oxidative stress damage this function of LCAT. These findings indicate that the measurement of esterification of cholesterol and of 24(S)OH-$C_{NE}$ in the cerebral interstitial fluid represents the activity of LCAT and therefore reflects two conditions of the CNS which are negatively correlated. These conditions are the ability to remove sterols, such as esters of fatty acids in lipoproteins addressed to the bloodstream, and oxidative stress, for which the LCAT activity represents a biosensor. In other words, in this patent, the measurement of 24(S)OH-CE in the CSF, a fluid containing molecules drained from the interstitial fluid of the CNS, is presented as a diagnostic criteria for neurodegenerative phenomena. This type of diagnosis is based on the quantitative analysis of 24(S)OH-$C_{TOT}$ and 24(S)OH-$C_{NE}$ and on the determination of the level of 24(S)OH-CE relative to the level of 24(S)OH-$C_{NE}$ (ratio) or to the level of 24(S)OH-$C_{TOT}$ (fraction or percentage) in the CSF. The biomarker of neurodegeneration forming the basis of the invention is therefore the activity of esterification of 24(S)OH-$C_{NE}$ in the CNS, expressed by the above-mentioned level. Since 24(S)OH-$C_{NE}$ accumulates in the CNS and, in particular, in the CSF due to the accumulation of cholesterol and due to oxidative stress, and since these conditions represent early and neurotoxic events, the biomarker is clearly altered even in the initial phases of the disease before the symptoms are evident. In fact, the enzymatic activity depends on the substrate concentration, besides oxidative stress. The level of the biomarker decreases in neurodegenerative conditions and, since the level of 24(S)OH-CE transported in the bloodstream is not significantly modified, can also be determined in the blood plasma or in the blood serum. Therefore it must be understood that the present invention does not relate to the level of 24(S)OH-$C_{TOT}$ in the CSF or in the blood plasma or in the blood serum, but it relates to the level of 24(S)OH-CE in the CSF or in the blood plasma or in the blood serum.

Cholesterol is also esterified by LCAT in the interstitial fluid of the CNS and, therefore, the quota of esterified cholesterol is also an indicator of enzymatic activity and can be determined in the CSF, as it has been published. By contrast with the biomarker expressed by the quota of 24(S)OH-CE, which can also be determined in the blood plasma or serum, the quota of esterified cholesterol, instead, cannot be determined in the blood plasma or serum for evaluating the activity of LCAT in the interstitial fluid of the CNS and in the CSF. In fact, a significant quantity of cholesterol, of hepatic or dietary origin, is esterified in the blood by the plasma form of LCAT, which is separate from the form of LCAT of the CNS due to the blood-brain barrier. This massive esterification of cholesterol in the plasma changes the ratio of esters to free forms of cholesterol of nervous origin transported from the CNS to the bloodstream.

The significance of the biomarker (defined here as "activity of esterification of 24(S)OH-$C_{NE}$" or "activity of production of 24(S)OH-CE" in the interstitial fluid) compared with that of the level of esterification of cholesterol in the interstitial fluid of the CNS lies not only in the opportunity to measure the level of 24(S)OH-CE also in the blood plasma or in the blood serum, but also in the fact that 24(S)OH-$C_{NE}$ is synthesised specifically in response to oxidative stress and the accumulation of cholesterol, these being two proven key events of the early phases of neurodegeneration. The biomarker therefore results from the increased availability of a specific enzymatic substrate, which, in addition to being neurotoxic *per se,* reflects an initial phase of the neurodegenerative process.

Since 24(S)OH-$C_{NE}$ and 24(S)OH-CE are transported from the interstitial fluid to the CSF and to the bloodstream, the analysis of the biomarker in the plasma or in the serum may substitute the analysis of the biomarker in the CSF, thus substituting the invasive sampling of CSF (by means of lumbar puncture) with a simple sampling of blood. The advantages of the blood sampling over lumbar puncture are clear, both from the point of view of the necessary technical expertise and the possible health consequences of the lumbar puncture, and from the point of view of the number and frequency of repetitions of the sampling procedures that are necessary for effective preventive medicine and for assessment of the clinical course of individual patients respectively.

The analysis of the biomarker, that is to say the measurements of the levels of 24(S)OH-$C_{NE}$ and 24(S)OH-$C_{TOT}$ in order to calculate the amount of 24(S)OH-CE and to determine the quota of 24(S)OH-CE (in terms of ratio with the level of 24(S)OH-$C_{NE}$, that is to say [24(S)OH-CE]/[24(S)OH-$C_{NE}$], or in terms of percentage, that is to say [24(S)OH-CE] $\times$ 100/[24(S)OH-$C_{TOT}$], or fraction of the total forms, that is to say [24(S)OH-CE]/[24(S)OH-$C_{TOT}$]), consists in a simple procedure with sequential steps. In the first step, deuterated 24(S)OH-$C_{NE}$, with internal standard function, is added to the CSF or to the plasma or to the serum. In the second step, the sample is subdivided into two aliquots and one of the two aliquots is saponified. In the third step, both the aliquots are extracted with hexane and the fraction of oxysterols is isolated from each organic extract by means of solid-phase extraction. In the fourth step, the oxysterols are separated by means of liquid chromatography, determining the quantity of 24(S)OH-C$_{TOT}$ with a mass spectrometry detector. In the fifth step, the amount of 24(S)OH-CE is calculated by subtracting from the amount of 24(S)OH-C$_{TOT}$ in the saponified sample the amount of 24(S)OH-C$_{NE}$ in the non-saponified sample. In the sixth and final step, the level of the biomarker is expressed as the quota of 24(S)OH-CE relative to the level of 24(S)OH-C$_{NE}$ or to the level of 24(S)OH-C$_{TOT}$ in percentage or fraction terms, as mentioned above.

The majority of applications of use of the biomarker concerns the diagnosis of the onset and of the rate of progression of neurodegeneration, the evaluation of the personalised response of the patient to therapy, and the development and validation of new drugs.

The advantages of the present invention lie in the identification of an early molecular biomarker of neurodegeneration, enabling therapeutic intervention before the symptoms of the disease are evident and making it possible to formulate the intervention itself on the basis of the individual response.

In addition, the advantages of the biomarker described in the present invention are those of reflecting significant and perhaps the greatest factors of neurodegeneration, that is to say the accumulation of cholesterol and oxidative stress in the CNS, and of being able to be analysed in a sample of plasma or serum, with a volume much smaller than that necessary for the analysis of CSF. In fact, the sensitivity of the analysis procedure described here makes it possible to obtain precise data with less than 0.1 mL of plasma or with little less than 1 mL of CSF.

An important advantage of the biomarker described in the present invention is that of having a predictive value of the onset of the neurodegenerative disease or a prognostic value of the progression of the disease itself, because the level of 24(S)OH-C$_{NE}$ (substrate of LCAT for the production of 24(S)OH-CE) hardly increases the cholesterol starting to accumulate in the CNS and above all in response to oxidative species in this same compartment. As reported in the present invention, the level of 24(S)OH-CE decreases when the catalytic efficacy of the enzyme decreases. This decrease of the level of 24(S)OH-CE occurs in spite of a rise of the synthesis of 24(S)OH-C$_{NE}$, which is known to be induced specifically by oxidative stress. In fact, it has been demonstrated that, in the condition of MCI, that is to say in an early phase of neurodegeneration in AD, the amount of 24(S)OH-C$_{TOT}$ increases in the CSF [45, 144].

The advantage of the biomarker described in the present invention (activity of LCAT or level of esterification of 24(S)OH-C$_{NE}$ in the CNS) and that can be measured in the CSF or in the plasma, is greater than the advantage of measuring the level of 24(S)OH-C$_{TOT}$ in the plasma, described in a published work [144]. In fact, the level of 24(S)OH-C$_{TOT}$ in the plasma reflects only the accumulation of cholesterol in the CNS, but does not provide information concerning the redox state in the CNS. In addition, it may be doubtful that the level of 24(S)OH-C$_{TOT}$, whether in the CSF or in the plasma, may unequivocally reflect the accumulation of cholesterol in the CNS of subjects subjected to treatment with statins. In fact, it is well known that statins reduce the synthesis of cholesterol not only in the liver but also in the CNS, and therefore subjects following therapy with statins may have reduced levels of cholesterol in the CNS and, consequently, reduced levels of plasma 24(S)OH-C$_{TOT}$ even in conditions of neurodegeneration. Instead, the esterification of 24(S)OH-C$_{NE}$ in the cerebral interstitial fluid and in the CSF (which contains molecules drained from the former) is an enzymatic process which is reduced in the presence of oxidised enzyme. In fact, the amount of active enzyme, decreased as a result of oxidative damage, produces a lesser quantity of 24(S)OH-CE, even if the level of the substrate 24(S)OH-C$_{NE}$ is normal or below the normal level.

Such advantages are therefore those that make it possible, with methodologies different for sensitivity and identification ability, to measure the above-mentioned biomarker so as to consider the response to the therapeutic treatment and that therefore make it possible to prevent effects caused by pharmacological overdosing and/or prolonged individual therapy with last-generation drugs and also to develop new drugs as discussed hereinafter. These advantages are directed to the market of clinical medicine and to that of the pharmaceutical industry.

A further advantage, directed to the market of the pharmaceutical industry, is the identification of LCAT as a key protein in the process of massive transport of cholesterol and of 24(S)OH-C$_{TOT}$ to the bloodstream. This protein, in so far as the structure and catalytic function thereof are altered during neurodegeneration, has proven to be a biosensor of the redox state of the CNS and a potential therapeutic target, and offers the pharmaceutical industry the opportunity to develop new drugs to stimulate esterification of cholesterol and of 24(S)OH-C$_{NE}$ based on peptide positive effectors, such as, but not exclusively, mimetic peptides of ApoE and/or ApoA-I and/or stimulators of the astrocyte secretion of ApoE and/or LCAT and/or organic stimulators of the activity of LCAT, such as, but not exclusively, the *compound A*. In addition, the pharmaceutical industry may take advantage of the use of the biomarker defined in the present invention to evaluate the efficacy of drugs based on transport systems, directed selectively (drug delivery system) to the CNS, of natural antioxidants (for example, but not exclusively, tocopherol or retinol) or synthetic antioxidants (for example, but not exclusively, bis[1-hydroxy-2,2,6,6-tetramethyl-4-piperidinyl] decantionate). It is known that stimulators of the activity or gene expression of some proteins regulated directly or indirectly by 24(S)OH-C such as, but not exclusively, NOS (nitric oxide synthase) or sPLA2-AII (secreted phospholipase A2, type AII) or ACAT (acylCoA-cholesterol acyltransferase) or ABC transporters (ATP binding Cassette) and EET2 transporters (glutamate transporter) or agonists of ROR (retinoic acid receptor-related orphan receptors) or LXR (liver X receptor) or PXR (pregnane X receptor), can limit the neurotoxicity of 24(S)OH-C$_{NE}$. It is known that effectors of CYP46A1, the enzyme which converts cholesterol into 24(S)OH-C$_{NE}$, or repressors of the expression of the gene *cyp46A1* can limit this neurotoxicity and that treatment with statins has a positive role in AD. The efficacy of all molecules as mentioned above by way of non-limiting examples, which antagonise the role of 24(S)OH-C$_{NE}$ on specific proteins or prevent the accumulation of cholesterol in the CNS, can be evaluated by monitoring the level of the biomarker described in the present invention, and the use of the biomarker for these purposes is therefore to be considered an integral part of the present patent.

The present invention therefore relates to a method for the diagnosis and/or prognosis of a neurodegenerative disease and/or for the monitoring of the efficacy of a therapeutic treatment of a neurodegenerative disease and/or for the screening of a therapeutic treatment of a neurodegenerative disease comprising the step of measuring the amount of esterified cerebrosterol [24(S)OH-CE] in an isolated biological sample from a subject and comparing the same with a proper value from a control sample.

The expression "measuring the amount of esterified cerebrosterol [24(S)OH-CE]" can also be intended as measuring the amounts of total cerebrosterol [24(S)OH-C$_{TOT}$] and of non-esterified cerebrosterol [24(S)OH-C$_{NE}$] and calculating the amount of [24(S)OH-CE] according to the following formula: [24(S)OH-CE] = [24(S)OH-C$_{TOT}$]- [24(S)OH-C$_{NE}$].

The amount of esterified cerebrosterol [24(S)OH-CE] can also be expressed as [24(S)OH-CE]/[24(S)OH-C$_{NE}$] or [24(S)OH-CE]/[24(S)OH-C$_{TOT}$] ratios, as the percentage [24(S)OH-CE] × 100/[24(S)OH-C$_{TOT}$] or as other indices of esterification of 24(S)OH-C$_{NE}$.

In a preferred aspect, the method according to the invention comprises the steps of:

a) measuring the amounts of total cerebrosterol [24(S)OH-C$_{TOT}$] and of non-esterified cerebrosterol [24(S)OH-C$_{NE}$];
b) calculating the amount of [24(S)OH-CE] according to the following formula:

$$[24(S)OH\text{-}CE] = [24(S)OH\text{-}C_{TOT}]\text{- }[24(S)OH\text{-}C_{NE}];$$

c) comparing the obtained value as in step b) with the proper value obtained from a control sample.

In the method according to the invention, a predetermined amount of deuterated 24(S)OH-C$_{NE}$ is preferably introduced, as an internal standard, into the isolated biological sample. Other proper internal standard may be used according to the knowledge of the skilled person. Preferably, the method according to the invention comprises the steps of:

a) introducing a predetermined amount of deuterated 24(S)OH-C$_{NE}$, as an internal standard, into the isolated biological sample;
b) separating said sample into a first and a second aliquot;
c) treating said first aliquot by means of saponification;
d) optionally, extracting and isolating 24(S)OH-C$_{NE}$ from each of said first and second aliquot;
e) measuring the amount of 24(S)OH-C$_{NE}$ in said first and said second aliquot and standardising obtained values with the measurement of the amounts of the internal standard, wherein the amount of 24(S)OH-C$_{NE}$ in the first aliquot corresponds to [24(S)OH-C$_{TOT}$] and the amount of 24(S)OH-C$_{NE}$ in the second aliquot corresponds to [24(S)OH-C$_{NE}$];
f) calculating the amount of [24(S)OH-CE] according to the formula [24(S)OH-CE] = [24(S)OH-C$_{TOT}$]- [24(S)OH-C$_{NE}$];
g) optionally calculating the ratio [24(S)OH-CE]/[24(S)OH-C$_{NE}$] and/or [24(S)OH-CE]/[24(S)OH-C$_{TOT}$], and/or the percentage [24(S)OH-CE] × 100/[24(S)OH-C$_{TOT}$];
h) comparing values as obtained in f) or g) with proper values obtained from a control sample.

Said amount of [24(S)OH-CE] is preferably related to the activity of the lecithin-cholesterol acyltransferase (LCAT) in the central nervous system.

In a preferred form, the neurodegenerative disease is selected from the group consisting of: Alzheimer's disease, multiple sclerosis, amyotrophic lateral sclerosis, Parkinson's disease or Huntington's disease.

In the method according to the invention, the biological sample is preferably selected from the group consisting of: serum, plasma, CSF, saliva, urine or fluid of the hair bulb. The biological sample may also be any other fluid in which 24(S)OH-C$_{NE}$ can be detected and measured in saponified and non-saponified aliquots.

The amount of 24(S)OH-C$_{NE}$ is preferably measured by means of specific antibody or coulometric or electrochemical detector.

A further object of the invention is the use of the biomarker esterified cerebrosterol [24(S)OH-CE] in an in vitro method for the diagnosis and/or prognosis of a neurodegenerative disease and/or for the monitoring of the efficacy of a therapeutic treatment of a neurodegenerative disease and/or for the screening of a therapeutic treatment of a neurodegenerative disease, said method being preferably as above defined.

Said biomarker can also be expressed as [24(S)OH-CE]/[24(S)OH-C$_{NE}$] ratios or [24(S)OH-CE]/[24(S)OH-C$_{TOT}$], as the percentage [24(S)OH-CE] $\times$ 100/[24(S)OH-C$_{TOT}$] or as other indices of esterification of 24(S)OH-C$_{NE}$.

In addition to the biomarker esterified cerebrosterol [24(S)OH-CE], also the level of the activity of the enzyme LCAT might represent a biomarker of the disease.

Another object of the invention is the use of esterified cerebrosterol 24(S)OH-CE] to determine the ratios [24(S)OH-CE]/[24(S)OH-C$_{NE}$] or [24(S)OH-CE]/[24(S)OH-C$_{TOT}$] or of the percentage [24(S)OH-CE] $\times$ 100/[24(S)OH-C$_{TOT}$] for the diagnosis and/or prognosis of a neurodegenerative disease and/or for the monitoring of the efficacy of a therapeutic treatment of a neurodegenerative disease and/or for the screening of a therapeutic treatment of a neurodegenerative disease and/or for measurement of the activity of lecithin-cholesterol acyltransferase (LCAT).

Said neurodegenerative disease is preferably selected from the group consisting of: Alzheimer's disease, multiple sclerosis, amyotrophic lateral sclerosis, Parkinson's disease and Huntington's disease.

A further object of the invention is the use of a kit comprising:

- means to measure the amount of at least one biomarker as above defined and optionally
- control means

for working the methods for the diagnosis and/or prognosis of a neurodegenerative disease and/or for the monitoring of the efficacy of a therapeutic treatment of a neurodegenerative disease and/or for the screening of a therapeutic treatment of a neurodegenerative disease, as defined above.

The expression "measuring the amount of at least one biomarker" can also be intended as measuring the amounts of total cerebrosterol [24(S)OH-C$_{TOT}$] and of non-esterified cerebrosterol [24(S)OH-C$_{NE}$] and calculating the amount of [24(S)OH-CE] according to the following formula: [24(S)OH-CE] = [24(S)OH-C$_{TOT}$]- [24(S)OH-C$_{NE}$], and said amount can also be expressed as the ratio [24(S)OH-CE]/[24(S)OH-C$_{NE}$] and/or [24(S)OH-CE]/[24(S)OH-C$_{TOT}$], and/or the percentage [24(S)OH-CE] $\times$ 100/[24(S)OH-C$_{TOT}$]. Therefore, the above means to measure the amount also comprise means to measure the amount of [24(S)OH-C$_{TOT}$] and/or [24(S)OH-C$_{NE}$].

Control means can be used to compare the amount or the increase of amount of the biomarker to a proper value from a control sample. The proper value may be obtained for example, with reference to known standard, either from a normal subject or from normal population.

The means to measure the amount of at least one biomarker as above defined are preferably at least one antibody, functional analogous or derivatives thereof.

Said antibody, functional analogous or derivatives thereof are specific for said biomarker.

In a preferred embodiment, the kit of the invention comprises:

- a solid phase adhered antibody specific for said biomarker;
- detection means of the ligand specific-biomarker complex.

The kits according to the invention can further comprise customary auxiliaries, such as buffers, carriers, markers, etc. and/or instructions for use.

In the case of a method or a kit for the diagnosis and/or prognosis of a neurodegenerative disease, the proper value from a control sample (herein also identified as reference value) may be the value measured in a sample taken from a healthy patient or from a patient affected by another non-degenerative neurological disease. In the case of a method or a kit for monitoring the efficacy of a therapeutic treatment, the proper value from a control sample may by a value measured in a sample taken from the same subject before initiation of the therapy or taken at various times during the course of the therapy. In the case of a method or a kit for the screening of a therapeutic treatment of a neurodegenerative disease, the proper value from a control sample may be the average of the values measured in samples taken from subjects without treatment and from subjects treated with a substance that is to be assayed or from subjects treated with a reference treatment.

Said control sample may be a second aliquot separated from the test sample, wherein it has been preferably introduced a predetermined amount of deuterated 24(S)OH-C$_{NE}$, as an internal standard.

In the present invention, the expression "measuring the amount" is intended as measuring the amount or concentration or level of the respective molecule, preferably semi-quantitative or quantitative. If by comparing the measured amount of the biomarker with the proper value obtained from a control sample of the biomarker, the amount of said biomarker in the test sample isolated from the subject corresponds to a lower value, the subject may present the neurodegenerative disease or go towards an aggravation of the neurodegenerative disease. If by comparing the measured amount of the biomarker with the proper value obtained from a control sample of the biomarker, the amount of said biomarker in the test sample isolated from the subject corresponds to a similar or higher value, the subject may be not affected by the neurodegenerative disease or go toward an amelioration of the disease, respectively. Alternatively, the expression "measuring the amount" is intended as measuring the alteration of the molecule. Said alteration can reflect an increase or a decrease in the amount of the biomarker esterified cerebrosterol ([24(S)OH-CE]). A decrease can be correlated to an aggravation of the disease. An increase can be correlated to an amelioration of the disease or to recovery of the subject.

Measurement of a biomarker can be performed directly or indirectly. Direct measurement refers to the amount or concentration measure of the biomarker, based on a signal obtained directly from the molecule, and which is directly correlated with the number of molecules present in the sample. This signal - which can also be referred to as intensity signal - can be obtained, for example, by measuring an intensity value of a chemical or physical property of the biomarker. Indirect measurements include the measurement obtained from a secondary component (e.g., a different component from the gene expression product) and a biological measurement system (e.g. the measurement of cellular responses, ligands, "tags" or enzymatic reaction products).

The term "amount", as used in the description refers but is not limited to the absolute or relative amount of the biomarker, and any other value or parameter associated with the same or which may result from this. Such values or parameters comprise intensity values of the signal obtained from either physical or chemical properties of the biomarker, obtained by direct measurement, for example, intensity values in an immunoassay, mass spectroscopy or mass spectrometry or a nuclear magnetic resonance. Additionally, these values or parameters include those obtained by indirect measurement, for example, any of the measurement systems described herein.

The present invention is described with reference to non-limiting examples also illustrated in the following figures.

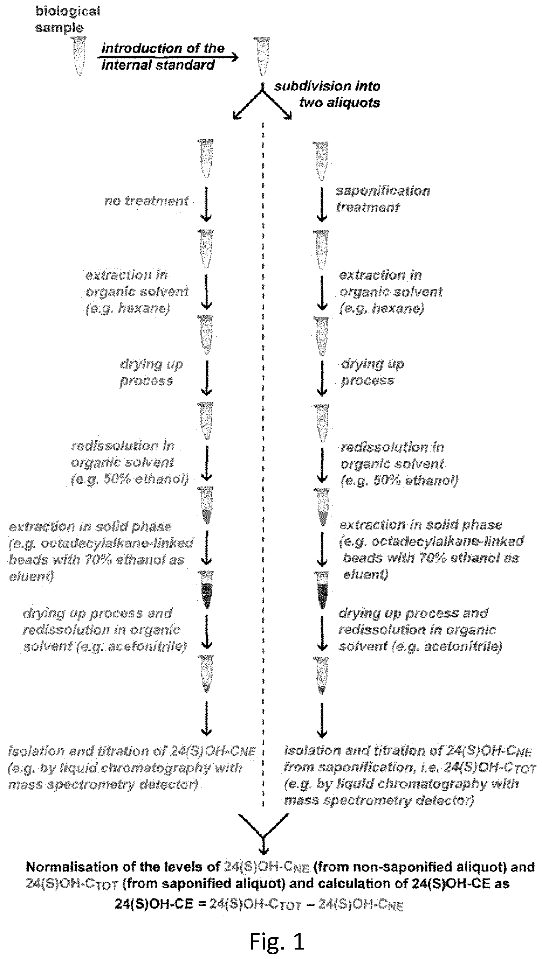

**Figure 1.** The steps summarizing the method used for titrating 24(S)OH-C$_{NE}$ and 24(S)OH-C$_{TOT}$, and for calculating the level of 24(S)OH-CE is outlined. Details are described in the text.

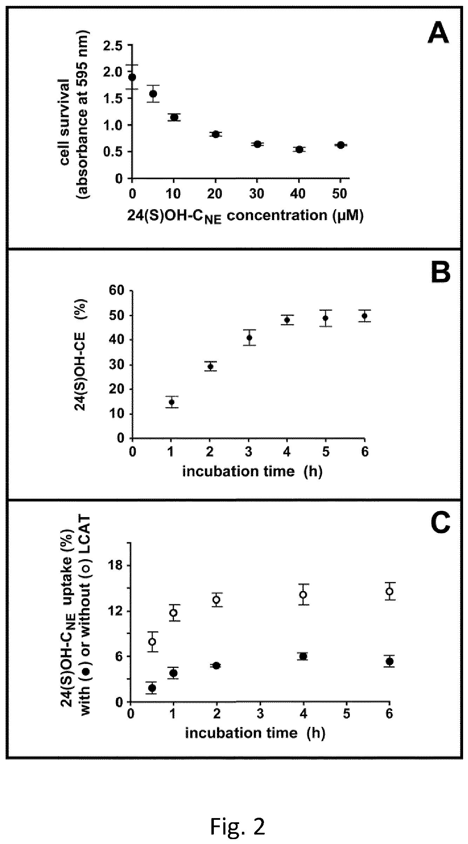

**Figure 2.** Box A. Cells from the SH-SY5Y cell line were differentiated by incubation with 10 $\mu$M of retinoic acid in DMEM containing 0.5% foetal bovine serum, 1% Pen-Strep, 1% L-glutamine, 1% of a mixture of factors known as "N2 medium supplement", essentially in accordance with a published procedure [146]. The incubation was performed for 3 days at 37°C in an atmosphere containing 5% CO$_2$ (37°C/5% CO$_2$). $7 \times 10^3$ cells were laid in each well of a microplate and then challenged with different concentrations of 24(S)OH-C$_{NE}$ and finally analysed in order to evaluate the survival of the cells, as described in Example 1. Each sample was analysed in three separate experiments. The data are expressed as means $\pm$ SD (SD = standard deviation). Box B. 20 $\mu$M $^3$H-24(S)OH-C$_{NE}$ (300,000 dpm/mL) was incubated with 250 ng/mL purified LCAT and proteoliposomes containing 2 $\mu$M ApoA-I and 266 $\mu$M phosphatidylcholine (ApoA-I/PC) in DMEM with 0.5% HSA for various periods of time (from 1 to 6 h) at 37°C/5% CO$_2$. After incubation, the esterified and non-esterified forms of oxysterol were extracted and analysed as described in Example 1. The level of esterification is indicated in terms of percentage of $^3$H-24(S)OH-CE. Each sample was analysed in three separate experiments. The data are expressed as means $\pm$ SD. Box C. Differentiated cells, as in the key of box A, were challenged with 20 $\mu$M $^3$H-24(S)OH-C$_{NE}$ (300,000 dpm/mL) previously treated or untreated, as described for box B, with LCAT and ApoA-I/PC for 5 h. After various incubation times (from 1 to 6 h), the cells were separated from the culture medium and lysed. The culture medium was analysed in order to measure any unincorporated radioactive material, whereas the lysate was analysed in order to measure any incorporated radioactive material (expressed as % of the sum of incorporated radioactive material plus unincorporated radioactive material). Experimental details are described in Example 1. White circles: incorporation of radioactive material incubated with the cells after pre-incubation without LCAT and ApoA-I/PC. Black circles: incorporation of radioactive material incubated with the cells after pre-incubation with LCAT and ApoA-I/PC. Each sample was analysed in three separate experiments. The data are expressed as means $\pm$ SD.

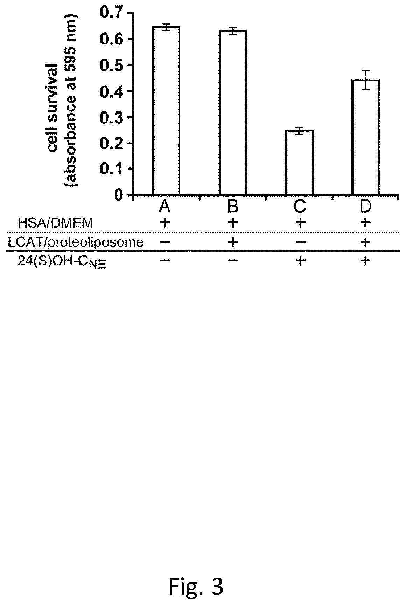

**Figure 3.** Differentiated cells, as in the key of Figure 2 (box A), were incubated with culture medium alone (sample A) or with 24(S)OH-C$_{NE}$ alone in culture medium (sample C) or with LCAT and ApoA-I/PC in culture medium (Sample B) or with a mixture of 24(S)OH-C$_{NE}$, LCAT and ApoA-I/PC in culture medium (sample D), as described in the key of Figure 2 (box B). The composition of the various culture media is shown below the histogram. Cellular survival after 5 h of incubation at 37°C/5% CO$_2$, was evaluated as described in Example 1. Each sample was analysed in three separate experiments. The data are expressed as means $\pm$ SD.

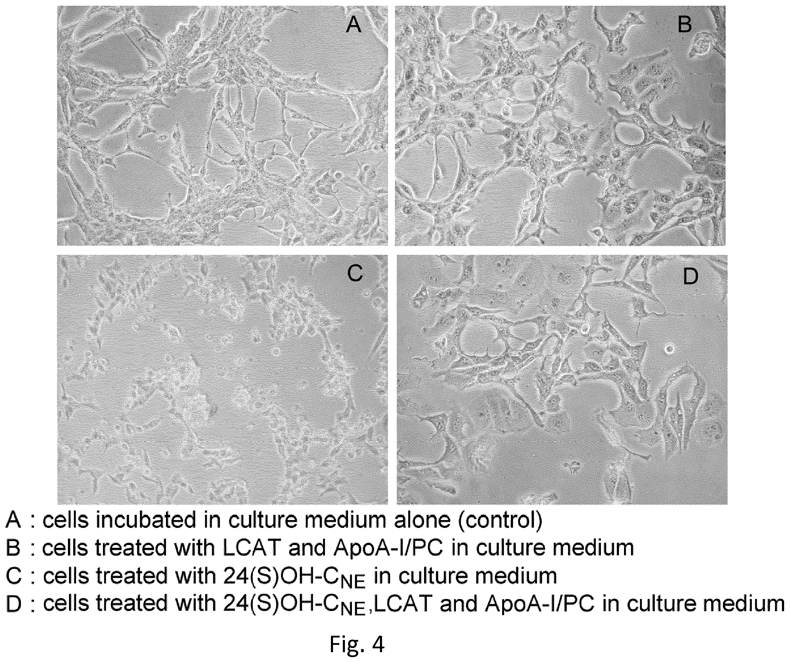

**Figure 4.** Images acquired by optical microscope (magnification: $10 \times 20$) of the cells incubated with the four different culture media described in the key of Figure 3. The letters A, B, C and D (which label the images) correspond respectively to samples A, B, C and D shown in Figure 3.

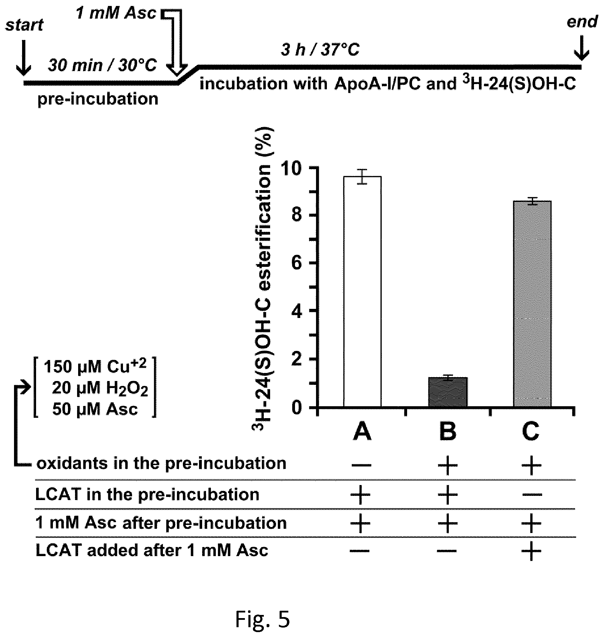

**Figure 5.** An outline of the process to which three samples have been subjected is shown at the top. At the bottom, the components of each sample are shown in a table. The oxidants were formed by a mixture of 150 $\mu$M CuCl$_2$, 50 $\mu$M Asc and 20 $\mu$M H$_2$O$_2$. Purified LCAT (250 ng/mL) was pre-incubated and added to the culture medium for cellular incubation (containing ApoA-I/PC) as indicated in the table at the bottom. 1 mM Asc was used to separate the pre-incubation from the incubation in the processes to which the samples were subjected. Each sample was analysed in three separate experiments. The data, expressed as means $\pm$ SD, were analysed using the Graph Pad Prism 3 program (Graph Pad Software; San Diego, CA, USA) in order to determine the significance of the difference of B from A and C ($P=10^{-5}$).

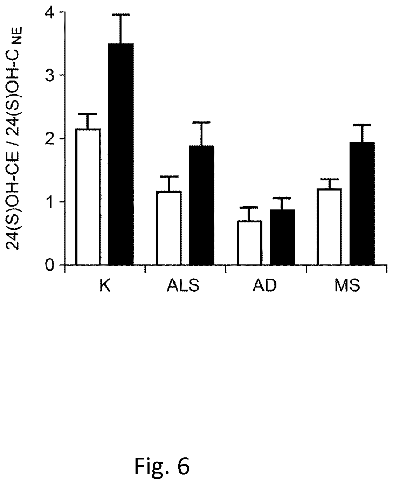

**Figure 6.** The [24(S)OH-CE]/[24(S)OH-C$_{NE}$] ratio was determined in the CSF (white bars) and in homologous plasma (black bars) of patients suffering from ALS (n=10), AD (n=10) and MS (n=10) and from control subjects (K, n=10), that is to say without neurodegeneration. The level of 24(S)OH-C$_{TOT}$ was measured in saponified samples and the level of 24(S)OH-C$_{NE}$ was measured in non-saponified samples, as described in Examples 3 and 4. The level of 24(S)OH-CE was calculated as the difference between the level of 24(S)OH-C$_{TOT}$ (that is to say 24(S)OH-C$_{NE}$ in the saponified sample) and that of 24(S)OH-C$_{NE}$ (in the non-saponified sample). The data, expressed as means ± SD, were analysed using the Graph Pad Prism 3 program in order to determine the significance of the differences (K compared with ALS, AD and MS: $P<0.01$).

## DETAILED DESCRIPTION OF THE INVENTION

## MATERIALS AND METHODS

*Reagents*

Human serum albumin (HSA), human apolipoproteins A-I and E (ApoA-I, ApoE3), Dulbecco Minimal Essential Medium (DMEM), L-glutamine, Crystal violet, sodium dodecyl sulphate (SDS), phosphatidylcholine, CuCl$_2$, ascorbic acid, 3-morpholino-sidnonimine (SIN-1), 2,2'-azino-bis(3-ethylbenzothiazoline-6-sulphonic acid (ABTS), hydrogen peroxide, KOH, NaCl and other chemicals were purchased from Sigma-Aldrich (St. Louis, MO, USA). Pen-Strep and "N2 medium supplement" were purchased from Gibco (Life Technologies; Grand Island, NY, USA). Organic solvents were purchased from Romil (Cambridge, UK). Mouse polyclonal IgG anti-LCAT was from Tebu-bio (Magenta, Italy; cat. # H00003931-A01). The human neuroblastoma cell line SH-SY5Y was kindly provided by the Institute of Genetics and Biophysics (Naples, Italy). Silica plates on a plastic substrate, for thin-layer chromatography (TLC), were purchased from Macherey-Nagel (Düren, Germany). 96-well polystyrene plates, for cell culture, and other disposable material from Becton Dickinson (Franklin Lake, NJ, USA) were used. 24(S)OH-C$_{NE}$ was purchased from Steraloids (Newport, RI, USA; cat. # C6496-000). $^3$H-24(S)OH-C$_{NE}$ [22-23 $^3$H] was purchased from American Radiolabeled Chemicals (St. Louis, MO, USA; cat. # 0783). $^2$H-24(S)OH-C$_{NE}$ [25,26,26,26,27,27,27 $^2$H] was purchased from Avanti Polar Lipids (Alabaster, A, USA; cat. # 700018). The BEH C18 nanoACQUITY column (1.7 μm, 150 μm × 100 mm) for UPLC was purchased from Waters (Milford, MA, USA). The cerebrospinal fluid and the homologous plasma were obtained, respectively, by means of lumbar puncture and blood sampling (in heparinised test tubes) from volunteers after informed consent about the use of the sampled fluids for research purposes. This material was provided by the Clinica delle Malattie Nervose e Mentali (Clinic of Nervous and Mental Diseases) of the Second University of Naples.
*Method for determining the levels of 24(S)OH-C$_{NE}$, 24(S)OH-C$_{TOT}$, and 24(S)OH-CE in*

*biological samples*

The procedure requires successive operative steps, summarised as follows:

1) Introduction of an internal standard (for example $^2$H-24(S)OH-C) in the sample of CSF or blood plasma.
2) Division of the sample into two aliquots of equal or similar volume.
3) Alkaline treatment of one of the two aliquots in order to hydrolyse the ester bond of 24(S)OH-CE.
4) Extraction with organic solvent (for example hexane) of both aliquots, keeping them separate, thus obtaining two separate organic extracts.
5) Drying of the two separate organic extracts and redissolution of the two separate extracts in a suitable solvent (for example 50% ethanol).
6) Extraction of the two separate organic extracts in order to obtain two separate eluates. In particular, the extraction can be performed through solid phase. The solid phase is preferably octadecyl alkane linked to inert microparticles (equilibrated with 50% ethanol for the load of each organic extract). The eluent phase is preferably 70% ethanol.
7) Drying of the two separate eluates and redissolution of the two separate dried eluates with a suitable solvent, preferably acetonitrile, in order to obtain two separate products.
8) Isolation of 24(S)OH-C$_{NE}$ from each separate product. The isolation is preferably carried out by means of "ultra performance liquid chromatography" (UPLC) associated with an electrical charge source and a mass spectrometry detector.
9) Measurement of the amount of 24(S)OH-C$_{NE}$ in each separate product. The amount of 24(S)OH-C$_{NE}$ in the aliquot subjected to alkaline hydrolysis treatment (saponified aliquot) is referred to as [24(S)OH-C$_{TOT}$], and the amount of 24(S)OH-C$_{NE}$ in the aliquot not subjected to alkaline treatment (non-saponified aliquot) is referred to as [24(S)OH-C$_{NE}$];

10) Normalisation, based on the detected amount of internal standard, of the amounts of 24(S)OH-$C_{NE}$ measured in each separate product and calculation of the amount of [24(S)OH-CE] ([24(S)OH-CE] = [24(S)OH-$C_{TOT}$] - [24(S)OH-$C_{NE}$]) and of [24(S)OH-CE]/[24(S)OH-$C_{NE}$] or [24(S)OH-CE]/[24(S)OH-$C_{TOT}$] or [24(S)OH-CE] $\times$ 100/[24(S)OH-$C_{TOT}$].

All of the above-described steps of the procedure, outlined in **Figure 1,** were used by various research groups in order to measure 24(S)OH-$C_{TOT}$ or the percentage of 24(S)OH-CE in the CSF or in the plasma of normal individuals.

In a first variant of the present invention, radioactive 24(S)OH-$C_{NE}$ can be used as an internal standard.

In a second variant, any molecule having characteristics in terms of mass and solubility in aqueous solution which allow it to be fractionated together with 24(S)OH-$C_{NE}$ in the various steps of the preparation procedure for chromatography and having chromatographic elution times close to, but clearly different from, that of 24(S)OH-$C_{NE}$ can be used as an internal standard. For example, some steroids or various types of other organic molecules, synthetic or natural (but not present in the human species), can be used as an internal standard since they meet the above-mentioned conditions.

In a third variant, the extraction in solid phase can be developed with normal phase instead of with reverse phase mode. In addition, the solid phase can be formed by various types of hydrophobic molecules bound to (or as integral part of) inert support microparticles.

In a fourth variant, the detector of the various types of molecules that can be fractionated with UPLC or HPLC may be an electrochemical detector. In this case, a suitable amount of radioactive 24(S)OH-$C_{NE}$ (to be measured using liquid scintillation techniques after having isolated and collected the fractions containing 24(S)OH-$C_{NE}$, obtained at the end of the treatment of the two initial aliquots) or an amount of another hydroxycholesterol, which is normally present at levels far below those of 24(S)OH-$C_{TOT}$ and is added to the sample in a concentration much greater than that expected for 24(S)OH-$C_{TOT}$, can be used as an internal standard. By way of non-limiting example, the oxysterol 25-hydroxycholesterol (which has plasma levels similar to 1/10 of those of 24(S)OH-$C_{TOT}$) can be added with a final concentration 50-100 times greater than its concentration in the plasma so as to thus minimise the standard error caused by the partial presence of its esters with fatty acids (saponifiable esters) in the plasma. This variant, based on the use of the electrochemical detector, must be considered as fully justified by the fact that the mass spectrometry analysis used as described in the present invention may have the limitation of equipment that is more costly and requires greater technical expertise and maintenance.

In a fifth variant, the amount of 24(S)OH-$C_{NE}$ derived, separately, from the samples of saponified and non-saponified CSF or plasma can be measured by means of immunochemical assays, such as, but not exclusively, that based on the ELISA procedure and sold by Enzo Life Sciences (cat. # ADI-900-210).

Other chemical or biochemical procedures can be used to hydrolyse 24(S)OH-CE and then measure the levels of 24(S)OH-$C_{NE}$ for the purposes of determining the [24(S)OH-CE]/[24(S)OH-$C_{NE}$] ratio or other indices of esterification of 24(S)OH-$C_{NE}$ in the CSF or in the blood plasma. The esterification levels of 24(S)OH-$C_{NE}$ in the blood plasma are identical or very similar to those in the blood serum. A further variant fully included within the scope of the present invention therefore consists in using any method and equipment able to detect the amount of 24(S)OH-CE, in absolute terms or terms relative to the amount of 24(S)OH-$C_{NE}$ or 24(S)OH-$C_{TOT}$ in the CSF, in the blood plasma or serum and/or other types of bodily fluids.

It is also understood that the above-mentioned forms of 24(S)OH-CE and 24(S)OH-$C_{NE}$ or 24(S)OH-$C_{TOT}$ are detected by means of any procedure, even a procedure different from those described in the present invention, performed on the bodily fluids of individuals suffering from neurodegeneration, the measurement and use of the biomarker (even in an algorithm comprising parameters concerning analysis of images or specific alleles or other biomarkers) being understood to be an integral part of the present invention.

**EXAMPLES**

With the objective of ascertaining whether the activity of LCAT in the CNS can reflect pathological neurodegeneration conditions, possibly associated with oxidative stress or altered stimulation by various molecular effectors in this anatomical compartment, and whether the measurement of the level of esterification of 24(S)OH-$C_{NE}$ in the CSF and in the plasma may constitute a diagnostic criterion of the activity of LCAT in the CNS, steps were carried out in accordance with the following outline:

1 - demonstration (*proof-of-concept*) that the activity of LCAT, converting 24(S)OH-$C_{NE}$ into esters with fatty acids (24(S)OH-CE), prevents or limits the toxic effect of 24(S)OH-$C_{NE}$ in cultures of neuronal cells;

2 - demonstration (*proof-of-concept*) that oxidative stress causes low levels of the biomarker, that is to say reduces the activity of esterification of 24(S)OH-$C_{NE}$ by LCAT;

3 - analysis of the level of esterification of 24(S)OH-$C_{NE}$ in the CSF of patients suffering from neurodegenerative diseases (MS, AD, ALS) and of control individuals;

4 - verification (*proof-of-concept*) that the ratio of the amount of esterified form to the non-esterified form of cerebrosterol (24(S)OH-CE/24(S)OH-$C_{NE}$) or the ratio of the amount of the esterified form to the total amount of esterified and non-esterified forms of cerebrosterol ([24(S)OH-CE]/[24(S)OH-$C_{TOT}$]) or the percentage of esterified cerebrosterol ([24(S)OH-CE $\times$ 100]/[24(S)OH-$C_{TOT}$]) in the CSF is positively linked to 24(S)OH-CE/24(S)OH-$C_{NE}$ or 24(S)OH-CE/[24(S)OH-$C_{TOT}$] or the percentage of 24(S)OH-CE present in the plasma.

These steps will be described in detail hereinafter.

**EXAMPLE 1: Step 1** Demonstration (*proof-of-concept*) that the activity of LCAT, converting 24(S)OH-$C_{NE}$ into esters with fatty acids (24(S)OH-CE), prevents or limits the toxic effect of 24(S)OH-$C_{NE}$ in cultures of neuronal cells.

The oxysterol 24(S)OH-$C_{NE}$, at concentration toxic for SH-SY5Y neuronal cells (neuroblastoma line), differentiated with retinoic acid in accordance with a published procedure [146], was incubated with LCAT in culture medium and then tested with the neurons in order to evaluate the cell survival thereof. This step was developed first by evaluating the survival of the cells as a function of the concentration of 24(S)OH-$C_{NE}$ in the culture medium (Step 1A), then by finely tuning the incubation time necessary to obtain maximum incorporation of 24(S)OH-$C_{NE}$, previously treated or not with LCAT, in the cells (Step 1B), and lastly measuring the survival of the cells, incubated for this period with a suitable concentration of 24(S)OH-$C_{NE}$, previously treated or not with LCAT (Step 1C).

In Step 1A the differentiated cells (7 $\times$ 10$^3$ adhered for each well of a microplate for cell culture) were first treated for 5 h at 37°C/5% $CO_2$ with different concentrations of 24(S)OH-$C_{NE}$ (from 5 to 50 $\mu$M) in culture medium, formed by DMEM and 0.5% HSA. Then, after substitution of this medium with fresh medium (but devoid of 24(S)OH-$C_{NE}$), the cells were incubated further for 16 h at 37°C/5% $CO_2$. The analysis of cell survival was performed after removal of the culture medium. For this analysis, the cells were fixed with 1% glutaraldehyde in PBS (PBS: 137 mM NaCl, 10 mM $Na_2HPO_4$, 2 mM $KH_2PO_4$, pH 7.4) and then treated for 30 min with a solution of 0.1% *Crystal Violet* in water, at room temperature. After removal of this solution and after washing with water, the stain incorporated in the viable cells was solubilised with 10% acetic acid in water in order to measure the amount of said cells (as an index of cell survival) in terms of absorbance at 595 nm. Only 58% and 43% of the cells survived after incubation, respectively, with 10 and 20 $\mu$M 24(S)OH-$C_{NE}$ (**Figure 2, box A**). The data confirmed that 24(S)OH-$C_{NE}$ is neurotoxic in neuron culture and indicated that the concentration thereof of 20 $\mu$M could be used to develop Step 1B and Step 1C.

In Step 1B, 20 $\mu$M of $^3$H-24(S)OH-$C_{NE}$ (300,000 dpm/mL) was pre-incubated in culture medium (DMEM containing 0.5% HSA) in the presence or absence of 250 ng/mL LCAT, purified in accordance with a published procedure [147], and proteoliposomes containing ApoA-I and phosphatidylcholine (ApoA-I/PC), prepared in accordance with a published procedure [148] omitting cholesterol. The concentrations of ApoA-I and phosphatidylcholine, associated with the proteoliposomes, were 2 $\mu$M and 266 $\mu$M respectively in the culture medium. After 5 h of pre-incubation at 37°C/5% $CO_2$, 49.3$\pm$2.8 % (mean $\pm$ SD) of $^3$H-24(S)OH-$C_{NE}$ was found esterified by LCAT (**Figure 2, box B**). In particular, the amount of esterified $^3$H-24(S)OH-$C_{NE}$ (that is to say $^3$H-24(S)OH-CE) was measured as detailed hereinafter. The lipids, extracted with hexane from each pre-incubated mixture and then dried, were solubilised with 50 $\mu$L of $CHCl_3$ and the resulting solutions were separately loaded on a silica plate for TLC. The chromatography process was carried out using ethyl acetate/hexane (30/70, v/v). The stationary phase regions harbouring $^3$H-24(S)OH-CE (Rf=0.70-0.75) and $^3$H-24(S)OH-$C_{NE}$ (Rf=0.25-0.35) were separately removed and then analysed for the radioactivity content thereof by liquid scintillation using the Packard TriCarb 2100TR counter (Global Medical Instrumentation, Ramsey, MI, USA). It is worth to mention that, when (in control experiments) the mixtures contained 6% (v/v) anti-LCAT antibodies, $^3$H-24(S)OH-CE was not produced after any pre-incubation time. Culture media pre-incubated for 5 h, as first described, were then added to the differentiated cells (7 $\times$ 10$^3$ per well, prepared as mentioned above) in order to measure the amount of $^3$H-24(S)OH-$C_{NE}$ and $^3$H-24(S)OH-CE incorporated during subsequent incubation at 37°C/5% $CO_2$, as detailed hereinafter. After different incubation times (from 0.5 to 6 h), the culture media with and without LCAT were removed and the cells were lysed with 0.1% SDS. Each lysate was then subdivided into two equal aliquots. One aliquot containing all the incorporated material was added directly to scintillation liquid in order to measure the radioactivity thereof using the counter mentioned above, whereas the other aliquot was stirred vigorously with 9 volumes of hexane and the organic phase was then analysed in order to measure the amount of $^3$H-24(S)OH-CE which was present in the cells. In particular, the residue of material, extracted from each aliquot with hexane and then dried, was solubilised with 50 $\mu$L of $CHCl_3$. The resulting solution was loaded on a silica plate for TLC, and the chromatography process was carried out using ethyl acetate/hexane (30/70, v/v). The stationary phase region harbouring $^3$H-24(S)OH-CE (Rf=0.70-0.75) was removed, and scintillation liquid was added in order to measure the radioactivity thereof. The results indicate that after 4 h of incubation 14% and 6% of $^3$H-24(S)OH-$C_{NE}$ was incorporated in the cultured cells, respectively, in the medium pre-incubated with $^3$H-24(S)OH-$C_{NE}$ without LCAT and proteoliposomes and in the medium pre-incubated with $^3$H-24(S)OH-$C_{NE}$ in the presence of LCAT and proteoliposomes. After this incubation time, reduced or no incorporation was found (**Figure 2,**

box C). In addition, it was observed that $^3$H-24(S)OH-CE was accumulated in the cells during incubation both with culture medium containing $^3$H-24(S)OH-C$_{NE}$ treated with LCAT plus proteoliposomes and with medium containing $^3$H-24(S)OH-C$_{NE}$ not treated with LCAT plus proteoliposomes. In particular, the percentage of $^3$H-24(S)OH-CE after 4 h of incubation was approximately 60% in the cells cultured in the medium pre-incubated with $^3$H-24(S)OH-C$_{NE}$ without LCAT and proteoliposomes, whereas it was approximately 50% in the cells cultured in the medium pre-incubated with $^3$H-24(S)OH-C$_{NE}$ but in the presence of LCAT and proteoliposomes. This experiment indicates that LCAT and proteoliposomes produce esters of 24(S)OH-C$_{NE}$ (that is to say 24(S)OH-CE) and limit the incorporation of 24(S)OH-C$_{NE}$ in cells. In addition, the experiment indicates that incorporated 24(S)OH-C$_{NE}$ is esterified within the cell, undoubtedly by the known activity of the enzyme acyl-CoA cholesterol acyltransferase (ACAT), and that the 24(S)OH-CE esters produced by LCAT are not incorporated, but remain restricted to the culture medium. Lastly, this experiment indicates that, under the used culture conditions, 5 h of incubation are more than sufficient in order to evaluate the effect of 24(S)OH-C$_{NE}$ on the cells.

In Step 1C, 20 $\mu$M 24(S)OH-C$_{NE}$ was pre-incubated with LCAT and proteoliposomes for 5 h, as described above. After the pre-incubation, the culture medium was added to the differentiated cells ($7 \times 10^3$ per well, prepared as mentioned above). After incubation for 5 h at 37°C/5% CO$_2$, the culture medium with 24(S)OH-C$_{NE}$ was changed for fresh culture medium, containing DMEM and 0.2 % HSA alone, and the cells were incubated for further 16 h at 37°C/5% CO$_2$. The treatment of the culture medium with LCAT, compared with a control treatment without LCAT, significantly reduced the toxic effect of 24(S)OH-C$_{NE}$ on the cells (**Figures 3 and 4**). This experiment, together with the experiments described previously, indicates that the enzyme limits the neurotoxic effect of 24(S)OH-C$_{NE}$, converting it into 24(S)OH-CE.

**EXAMPLE 2: Step 2** Demonstration (*proof-of-concept*) that oxidative stress causes low levels of the biomarker, that is to say reduces the activity of esterification of 24(S)OH-C$_{NE}$ by LCAT.

The purified enzyme LCAT was treated with an oxidative stress system and was then incubated with 24(S)OH-C$_{NE}$ in order to evaluate the efficiency of esterification of this oxysterol. This experiment was carried out after having finely tuned the oxidative stress system, assaying the effect of various concentrations of cupric ions (Cu$^{+2}$, from CuCl$_2$) alone or together with different concentrations of hydrogen peroxide (H$_2$O$_2$) or together with different combinations of concentrations of H$_2$O$_2$ and ascorbate (Asc) on the oxidation of the target of hydroxyl radicals 2,2'-azino-bis(3-ethylbenzothiazoline-6-sulfonic) acid, known by the acronym ABTS. The different concentrations of Cu$^{+2}$ and Asc used were within the ranges of values found in the CSF for these two ions, as reported in the literature [149-152], and the different concentrations of H$_2$O$_2$ were lower than values found to be non-toxic for nervous cells in culture [153-156]. ABTS is a chromogenic substance that can be converted into its radical monocationic form ABTS$^{\bullet+}$ if treated with an oxidative agent. The measurement of the absorbance of ABTS$^{\bullet+}$ (at 414 nm) has made it possible to evaluate that the mixture containing 150 $\mu$M CuCl$_2$/20 $\mu$M H$_2$O$_2$/50 $\mu$M Asc produces, at 30°C, oxidative species which effectively oxidise ABTS and that the addition of 1 mM Asc to the mixture prevents or blocks oxidation of the target. This oxidation mixture was therefore used to treat LCAT (250 ng/mL) at 30°C, and, after 30 min of treatment, 1 mM Asc was added in order to stop the oxidation of the enzyme. The solution with the treated enzyme was then incubated with 20 $\mu$M of $^3$H-24(S)OH-C$_{NE}$ in the presence of proteoliposomes (1 $\mu$M of ApoA-I and 133 $\mu$M of phosphatidylcholine) for 3 h at 37°C. After incubation, the lipids were extracted and fractionated by TLC in order to measure the activity of LCAT in terms of percentage of radioactive 24(S)OH-CE in the entire population of the radioactive species, separated by chromatography as described above. The incubation of the enzyme, added together with the proteoliposomes to the oxidation mixture after the addition of 1 mM Asc, was performed as a control. The incubation of the enzyme, in the absence of oxidation mixture, with the proteoliposomes was performed as a further control. The data obtained indicate that the oxidation mixture reduced the enzymatic activity to 15% (1.27 $\pm$ 0.10 vs 8.62 $\pm$ 0.15 nmol/h/ml in the controls with enzyme added to the mixture after addition of 1 mM Asc; means $\pm$ SD, P=10$^{-5}$) (**Figure 5**). This result clearly indicates that LCAT, when oxidised with factors that are present in the CSF, loses a large part of its activity of esterification of 24(S)OH-C$_{NE}$, stimulated by ApoA-I. Similar results were obtained using 150 $\mu$M Asc in the oxidation mixture or ApoE as a stimulator or the enzyme. It is known that the peroxynitrite ion may also degrade the function of LCAT and that the molecule *3-morpholino,sydnonimine* (SIN-1) is used largely to generate such an oxidising ion. Experiments concerning the oxidation of LCAT with SIN-1 were carried out in experimental conditions similar to those described above, but using 1 mM SIN-1 as oxidant, in 20 min of pre-incubation, and an antioxidant mixture (1 mM urate/0.25 mM Asc/0.25 mM cysteine) in order to stop oxidative stress. After incubation with the proteoliposomes (2 $\mu$M ApoA-I and 266 $\mu$M phosphatidylcholine), the enzyme resulted significantly limited, as found in the experiments with CuCl$_2$ and H$_2$O$_2$ described above, in terms of the activity of the esterification of 24(S)OH-C$_{NE}$ (12.77 $\pm$ 0.78 vs 19.11 $\pm$ 0.49 nmoles/h/mL in the controls with enzyme added to the mixture after addition of the antioxidant mixture; means $\pm$ SD, P=2.4$\times$10$^{-3}$).

It is known that in physiological conditions the amount of molecules (including LCAT) degraded in the CSF or eliminated from the CSF is balanced by the amount of newly synthesised molecules released from the cells (including LCAT, secreted by the astrocytes) in the CSF. In addition, the oxidative stress associated with pathological conditions occurs when the concentrations of the oxidative agents predominate over those of the antioxidants, thus resulting in the fact that the oxidative stress can degrade more molecules of LCAT than all those secreted by the astrocytes in the CSF. These considerations lead to the conclusion that, in oxidative stress conditions, the activity of LCAT may decrease to levels that do not allow esterification of 24(S)OH-$C_{NE}$ with the efficiency of normal esterification activity.

**EXAMPLE 3: Step 3** Analysis of the level of esterification of 24(S)OH-$C_{NE}$ in the CSF of patients suffering from neurodegenerative diseases (MS, AD, ALS) and of control individuals.

Patients suffering from neurodegenerative diseases and individuals presenting no symptoms of neurodegeneration (control individuals) were recruited in order to take a sample of 1 mL of CSF and 1 mL of homologous plasma. The diagnosis of MS was made by Kurtzke's method [157]. All the patients studied suffering from MS presented the relapsing-remitting form of the disease and a disability index (EDSS) between 5.5 and 7. The diagnosis of "probable" AD was made on the basis of the criteria proposed by the National Institute of Neurological and Communicative Disorders and Stroke - Alzheimer's Disease and Related Disorders Association (NINDCDS-ADRDA) [158]. This diagnosis, based on behavioural and anatomo-functional criteria, is defined here as "probable" because, as is known, it is not conclusive and there are no diagnostical methods which make it possible to confirm AD during life. The diagnosis of ALS was made after exclusion of other neurodegenerative diseases (by means of MRI and neurological tests), of other types of myopathies and of metabolic, degenerative, inflammatory, paraneoplastic or hereditary myelopathies by means of the criteria known as El Escorial Revisited [159]. The control individuals were subjected to lumbar puncture for anaesthetic interventions or for clinical analyses, that provided negative results for any diagnosis of nervous pathology, and did not present any symptoms of neurodegeneration. Samples of CSF and samples of homologous plasma were taken from four groups of subjects (MS, AD, ALS and controls) with blood-brain barrier intact. Each group was formed by 10 subjects aged between 50 and 70 years. The integrity of the blood-brain barrier [160] in all subjects studied was evaluated by measuring the concentration of albumin in the CSF and by expressing this as a percentage of that in the plasma. In particular, for all subjects studied, this percentage was less than 0.9%. The titration of 24(S)OH-$C_{NE}$ in the CSF was performed in accordance with a procedure widely used for the preparation of samples [38, 41], but was combined with the fractioning of the population of oxysterols by means of liquid chromatography, a technique which has the advantage that the molecules can be detected by mass spectrometry without having to be derivatised beforehand [161]. The procedure was based on the treatment of the samples as follows.

Each sample of CSF was mixed with 5 ng/mL deuterated 24(S)OH-$C_{NE}$ ($^2$H-24(S)OH-$C_{NE}$) and was then divided into two aliquots each measuring 500 $\mu$L. One aliquot, upon addition of 1.5 mL of 1 M KOH in ethanol, was saponified (by means of incubation for 1 h at 37°C) and the other was not. The lipids of both aliquots were prepared by means of two successive extractions, first with hexane and then with solid phase; of the two separate fractions of oxysterols obtained from the two separate aliquots, one contained 24(S)OH-$C_{TOT}$, that is to say the amount of original 24(S)OH-$C_{NE}$ (already present in the aliquot before saponification) plus the amount of 24(S)OH-$C_{NE}$ obtained by the saponification of 24(S)OH-CE, whereas the other one (originating from the non-saponified aliquot) contained 24(S)OH-$C_{NE}$ plus 24(S)OH-CE. Saponification, extraction with hexane and extraction in solid phase were performed under the conditions defined by publications by researchers of proven experimental qualification [38-41]. In brief, the extraction in solid phase was performed for each sample by first drying the lipids extracted with hexane, then solubilising them with 50% ethanol, then loading the hydroalcohol solution on an Isolute MFC18 column (Biotage, Uppsala, Sweden) equilibrated in 50% ethanol, and lastly by eluting the fraction of oxysterols with 70% ethanol. 24(S)OH-$C_{NE}$ was isolated from each fraction of oxysterols and was titrated by means of reverse-phase UPLC, with mass spectrometry detection (using $^2$H-24(S)OH-$C_{NE}$ as an internal standard for calibration). More specifically, each fraction of oxysterols, derived from the extraction in solid phase, was dried; then, the lipid residue was redissolved in acetonitrile and injected into the UPLC system (Accela 1250; Thermo Fisher Scientific, Cambridge, MA, USA); chromatography was developed at 1.5 $\mu$L/min and at room temperature with a mobile phase formed by two solutions (A: 0.1% HCOOH in water - B: 0.1% HCOOH in acetonitrile) mixed with a program (50% B from 0 to 5 min, linear gradient from 50 to 80% of B in 0.5 min, 80% B from 5.5 to 10 min); the effluent was analysed by means of mass spectrometry (Orbitrap Elite, interfaced with the column by means of a nanospray ionic source; Thermo Fisher Scientific; Cambridge, MA, USA). It was thus possible to obtain from the saponified aliquot the titre of 24(S)OH-$C_{TOT}$ in the CSF, whereas the titre of 24(S)OH-$C_{NE}$, which reflects the level of non-esterified forms in the CSF, was obtained from the non-saponified aliquot. Other procedures, similar to that described, can be used for titration both of 24(S)OH-$C_{TOT}$ and 24(S)OH-$C_{NE}$. The molar amount of 24(S)OH-CE in the CSF was calculated (by means of subtraction of the molar amount of 24(S)OH-$C_{NE}$ from the molar amount of 24(S)OH$_{TOT}$) in order to then determine the relative value thereof in terms of ratio [24(S)OH-CE]/[24(S)OH-$C_{NE}$] or [24(S)OH-CE]/[24(S)OH-$C_{TOT}$] or percentage of [24(S)OH-CE] in [24(S)OH-$C_{TOT}$]. The data obtained indicate that the levels of 24(S)OH-CE in the analysed control samples of CSF were greater than those in the samples of CSF from patients suffering from ALS, AD and MS **(Figure 6, white bars)**. In particular, the ratio [24(S)OH-CE]/[24(S)OH-$C_{NE}$] was 1.18±0.22, 0.73±0.23 and 1.22±0.16 respectively in the CSF of patients suffering from ALS, AD and MS, whereas the ratio was 2.15±0.25 in the CSF of the

controls (mean values $\pm$ SD). The differences between the ratio value in the CSF of the controls and the values found in the CSF of the patients are highly significant (P<0.001). The largeness of the SD depends on the fact that each group of patients was most probably composed by subjects in various phases of the disease. Similarly, the group of controls may have contained subjects that appeared to be normal but that had variable activity of LCAT, possibly influenced by events associated with ageing. The value of the ratio of [24(S)OH-CE]/[24(S)OH-C$_{NE}$] in the CSF of the controls was equivalent to 68% [24(S)OH-CE] in [24(S)OH-C$_{TOT}$], a value that is compatible with the information already published [45]. On the whole, the data collected concerning levels of 24(S)OH-CE in the CSF of the three groups of patients indicates clearly that the activity of LCAT in the CNS shows a deficit in the three types of neurodegeneration studied.

The deficit of activity of LCAT in the CSF of patients suffering from ALS, AD and MS could depend on various causes, for example insufficient stimulation by ApoE and/or ApoA-I, oxidative degradation of enzymatic function or a rise in the concentration of Hpt, the inhibitory effect of which on the enzyme *in vitro* is known [162]. In this context, it is expedient to mention that the haplotype ApoE4 stimulates LCAT to a lesser extent than the haplotypes ApoE2 and ApoE3 [163]. The levels of ApoE, ApoA-I and Hpt were measured using the ELISA procedure [82, 164, 165] in the CSF of each subject and yielded the following results (means $\pm$ SD) respectively: 2.03 $\pm$ 1.18 in ALS, 2.45 $\pm$ 0.92 in AD and 3.37 $\pm$ 1.96 $\mu$g/mL in MS (compared with 1.56 $\pm$ 1.16 $\mu$g/mL ApoE in the controls); 7.77 $\pm$ 3.45 in ALS, 4.84 $\pm$ 4.48 in AD and 6.6 $\pm$ 4.06 $\mu$g/mL in MS (compared with 3.77 $\pm$ 1.74 $\mu$g/mL ApoA-I in the controls); 2.45 $\pm$ 1.93 in ALS, 2.41 $\pm$ 0.48 in AD and 2.15 $\pm$ 1.09 $\mu$g/mL in MS (compared with 1.25 $\pm$ 0.85 $\mu$g/mL Hpt in the controls). No significant differences were found for each protein between patients and controls. The haplotype ApoE4 was sought using electrophoresis and immunochemical techniques [166-168] in the plasma of all subjects and was found only in one patient suffering from ALS and two patients suffering from AD (heterozygotes ApoE3/ApoE4). Since neither the low levels of ApoE and/or ApoA-I nor the higher levels of Hpt nor the presence of ApoE4 were found associated with lower levels of [24(S)OH-CE]/[24(S)OH-C$_{NE}$] in the CSF of the patients and of the controls, it is reasonable to assume that the functional alterations of the enzyme were caused primarily by oxidative degradation phenomena, although it cannot be ruled out that the expression of the gene for LCAT may be limited in the CNS of the patients. The phenomenon of the reduction of the amount of LCAT in the CSF of the patients seems to be unlikely however, since it is known that LCAT is secreted by the astrocytes, that the production of 24(S)OH-C$_{NE}$ increases neurodegeneration, and that 24(S)OH-C$_{NE}$ stimulates astrocyte secretion.

**EXAMPLE 4: Step 4** Verification (*proof-of-concept*) that the ratio of the amount of esterified form to the non-esterified form of cerebrosterol [24(S)OH-CE]/[24(S)OH-C$_{NE}$] or the ratio of the amount of esterified form to the total amount of esterified and non-esterified forms of cerebrosterol [24(S)OH-CE]/[24(S)OH-C$_{TOT}$] or the percentage of esterified cerebrosterol ([24(S)OH-CE] $\times$ 100/[24(S)OH-C$_{TOT}$]) present in the CSF is positively correlated with [24(S)OH-CE]/[24(S)OH-C$_{NE}$] or [24(S)OH-CE]/[24(S)OH-C$_{TOT}$] or the percentage of 24(S)OH-CE present in the plasma.

Samples of plasma measuring 1 mL, prepared from the blood from the same individuals from which the CSF was analysed, were mixed with 50 ng/mL of $^2$H-24(S)OH-C$_{NE}$. Each sample was then divided into two aliquots measuring 500 $\mu$L, of which one was subjected to alkaline hydrolysis and the other was not. The experimental procedure for titration of 24(S)OH-C$_{NE}$ in the saponified and non-saponified aliquots of each plasma sample was the same as described above for titration of this analyte in the saponified and non-saponified aliquots of the CSF samples. Similarly, the level of 24(S)OH-CE in each plasma sample was calculated from the levels of 24(S)OH-C$_{TOT}$ and 24(S)OH-C$_{NE}$. The data obtained indicate that the levels of 24(S)OH-CE in the analysed control plasma samples were greater than those in the plasma samples prepared from patients suffering from ALS, AD and MS (**Figure 6, black bars**). In particular, the ratio [24(S)OH-CE]/[24(S)OH-C$_{NE}$] was 1.82$\pm$0.36, 0.86$\pm$0.18, and 1.90$\pm$0.27 respectively in the plasma of patients suffering from ALS, AD and MS, whereas it was 3.50$\pm$0.44 in the plasma of the controls (mean values $\pm$ SD). The largeness of the SDs can be interpreted similarly to that of the SDs found for the ratio values in the CSF. The differences between the value of the ratio in the plasma of the controls and the values found in the plasmas of the patients are highly significant (P<0.001). In addition, the experimental data indicate that the ratio [24(S)OH-CE]/[24(S)OH-C$_{NE}$] in the plasma of the patients suffering from AD is considerably lower not only than that in the plasma of the controls (P<0.001), but also than the ratios in the plasmas of the patients suffering from ALS and MS (P<0.01).

The diagnostic sensitivity, specificity, precision, and accuracy (or efficiency) of the neurodegeneration analysis method were determined by discriminating the patients from the controls in a confusion matrix for various values of the ratio 24(S)OH-CE/24(S)OH-C$_{NE}$ in the plasma. The identification of true and false positives and true and false negatives at distinct reference values made it possible to verify that ratio values between 2.5 and 3.3 (that is to say values of the percentage 24(S)OH-CE $\times$ 100/24(S)OH-C$_{TOT}$ between 71 and 77%) can be used as a threshold of separation between patients and controls, respectively with values lower than and greater than this threshold. In particular, assuming 3 as the reference value of the ratio (that is to say 75% in terms of the percentage 24(S)OH-CE $\times$ 100/24(S)OH-C$_{TOT}$), the method presented sensitivity = 100%, specificity > 75%, precision > 88% and accuracy > 95% when diagnosing neurodegeneration in the overall population of the studied patients suffering from ALS, AD and MS . In addition, assuming 2.5 as the reference value of the ratio (that is to say 71% in terms of the percentage 24(S)OH-CE $\times$ 100/24(S)OH-C$_{TOT}$), the method has 100 % both for sensitivity and for specificity and also for precision and accuracy of diagnosis of AD.

The individual values of the ratio [24(S)OH-CE]/[24(S)OH-C$_{NE}$] in the CSF and in the homologous plasmas were analysed, and a positive correlation (r=0.95) was found, represented by the following equation:

$$y = 1.669\,x - 0.188$$

where y represents the ratio in the plasma and x represents the ratio in the CSF. This result indicates that the level of 24(S)OH-CE in the plasma reflects that in the CSF for each individual. Therefore, the measurement of the level of 24(S)OH-CE in the plasma can substitute that of the level of 24(S)OH-CE in the CSF in order to provide information concerning the activity of LCAT in the CNS. The fact that, in the plasma, the level of 24(S)OH-CE is greater for each individual than the level of 24(S)OH-CE in the CSF is most likely dependent on the increase of [24(S)OH-CE] produced by the plasma form of LCAT. However, such an increase (62.0, 56.3, 17.9 and 62.3%, respectively in subjects suffering from ALS, MS, AD and in the controls) does not prevent a positive correlation from being defined between the ratio [24(S)OH-CE]/[24(S)OH-C$_{NE}$] in the CSF and the ratio [24(S)OH-CE]/[24(S)OH-C$_{NE}$] in the plasma for each individual. Other considerations can be made on the basis of the fact that the above-mentioned equation exhibits a negative intercept on the ordinate axis and on the basis of the fact that the increase of the ratio [24(S)OH-CE]/[24(S)OH-C$_{NE}$] in the plasma of the patients suffering from AD, compared with the value found in the CSF of the same patients, is less than the increases of plasma ratios observed for the patients suffering from ALS and MS and for the controls.

The measurement of the level of 24(S)OH-CE in a wider population of plasma samples from patients suffering from AD (n=40) and from controls (n=40) confirmed that such a level is significantly lower in the patients than in the controls. In particular, the percentage 24(S)OH-CE $\times$ 100/24(S)OH-C$_{TOT}$ was $35.06\pm14.45$ in the plasma of the patients and $70.38\pm10.34$ in the plasma of the controls (P < 0.0001). Making account of all the data from plasma samples of patients suffering from AD and from plasma samples of the controls, and assuming a percentage value between 46 and 54% as the reference value, the method presented sensitivity > 89%, specificity > 97%, precision > 97%, and accuracy > 93% in diagnosing neurodegeneration.

Also plasma samples from subjects with MCI were analysed for their level of 24(S)OH-CE. In a period of one to eight years from the sampling, a part of these subjects displayed clear symptoms of AD (converters to AD; n= 8) whereas another part of these subjects did not (non-converters to AD; n=8). The percentage 24(S)OH-CE $\times$ 100/24(S)OH-C$_{TOT}$ was $41.37\pm15.31$ in the plasma of the converters and $77.43\pm15.45$ in the plasma of the controls (P = 0.0002). The sensitivity, the specificity, the precision, and the accuracy of the method were each over 88% when the percentage value of 60% was assumed as the reference value. These results indicate that the method discriminates converters from non-converters, and can be used for early diagnosis of neurodegeneration in AD.

Other steps and examples can describe procedures, including those that are ameliorative of technical aspects for various applications, for example, but not exclusively, as follows: the activity of LCAT is negatively correlated with oxidative stress indices (such as the level of groups of hydroxynonenals, nitrotyrosine and carbonyl) in the CSF; the amount of LCAT is positively correlated with oxidative stress indices, but is not correlated (either positively or negatively) with the level of 24(S)OH-CE in the CSF; the level of 24(S)OH-C$_{NE}$ (in saponified and non-saponified CSF samples) can be measured by means of gas chromatography with mass spectrometry detection or by means of liquid chromatography with electrochemical detection; the level of 24(S)OH-C$_{NE}$ (in saponified and non-saponified CSF or plasma samples) can be measured by means of the ELISA procedure with specific anti-24(S)OH-C$_{NE}$ antibodies.

## BIBLIOGRAPHIC REFERENCES

1- Ischiropoulos H. and Beckman J.S. J. Clin. Invest. 2003, 111:163-169.

2- Björkhem I. and Meaney S. Arterioscler. Thromb. Vasc. Biol. 2004, 24:806-815.

3- Halliwell B. J. Neurochem. 2006, 97: 1634-1658.

4- Okouchi M. et al. Antioxid. Redox Sign. 2007, 9:1059-1096.

5- Adibhatla R.A. and Hatcher J.F. Subcell. Biochem. 2008, 49:241-68.

6- Dietschy J.M. Biol. Chem. 2009, 390:287-293.

7- Amor S. et al. Immunology 2010, 129:154-169.

8- Glass C.K. et al. Cell 2010, 140:918-934.

9- Liu J.-P. et al. Mol. Cell. Neurosci. 2010, 43:33-42.

10- Shukla V. et al. Adv. Pharmacol. Sci. 2011, 2011:1-13.

11- Frears E.R. et al. Neuroreport 1999, 10:1699-1705.

12- Koudinov A.R. and Koudinova N.V. Clin. Med. Health Res. 2001; clinmed/2001100005 (vedi http://clinmed.netprints.org/cgi/content/full/2001100005v1)

13- Chauhan N.B. J. Lipid Res. 2003, 44:2019-2029.

14- Puglielli L. et al. Nat. Neurosci. 2003, 6:345-351.

15- Casserly I. and Topol E. Lancet 2004, 363:1139-46.

16- Cutler R.G. et al. Proc. Natl. Acad. Sci. USA 2004, 101:2070-2075.

17- Carter C.J. Neurochem. Int. 2007, 50:12-38.

18- Hirsch-Reinshagen V. and Wellington C.L. Curr. Opin. Lipidol. 2007,18:325-332.

19- Xiong H. et al. Neurobiol. Dis. 2008, 29:422-437.

20- Harris J.R. and Milton N.G.N. Cholesterol in Alzheimer's Disease and other Amyloidogenic Disorders. In: "Cholesterol Binding and Cholesterol Transport Proteins. Structure and Function in Health and Disease", series "Subcellular Biochemistry", Vol. 51, 2010, pp 47-75, Harris J.R. ed (Springer Verlag, Berlin, Germany).

21- Mathew A. et al. Brain Res. Bull. 2011, 86:1-12.

22- Ricciarelli R. et al. IUBMB Life 2012, 64:931-935.

23- Gamba P. et al. Ann. N.Y. Acad. Sci. 2012, 1259:54-64.

24- Pappolla M.A. et al. Free Radic. Biol. Med. 2002, 33:173-181.

25- Nelson T.J. and Alkon D.L. J. Biol. Chem. 2005, 280:7377-7387.

26- Gamba P. et al. Aging Cell 2011, 10:403-417.

27- Iannilli F. et al. Neurobiol. Aging 2011, 32:1033-1042.

28- Sodero A.O. et al. Neurobiol. Aging 2011, 32:1043-1053.

29- Wang Y. et al. J. Proteome Res. 2008, 7:1606-1614.

30- Brown A.J. and Jessup W. Mol. Aspects Med. 2009, 30:111-122.

31- Hirsch-Reinshagen V. et al. Mol. Cell. Biochem. 2009, 326:121-129.

32- Nieweg K. et al. J. Neurochem. 2009, 109:125-134.

33- Umeda T. et al. J. Neurosci. Res. 2010, 88:1985-1994.

34- Cartagena C.M. et al. Brain Res. 2010, 1319:1-12.

35- Pfrieger F.W. and Ungerer N. Progr. Lipid Res. 2011, 50:357-371.

36- Leoni V. and Caccia C. Biochimie 2013, 95:595-612.

37- Pitas R.E. et al. J. Biol. Chem. 1987, 262:14352-14360.

38- Dzeletovic S. et al. Anal. Biochem. 1995, 225:73-80.

39- Demeester N. et al. J. Lipid Res. 2000, 41: 963-974.

40- Xie C. et al. J. Lipid Res. 2003, 44:1780-1789.

41- Lütjohann D. et al. Proc. Natl. Acad. Sci. USA 1996, 93:9799-9804.

42- Björkhem I. J. Intern. Med. 2006, 260:493-508.

43- Russell D.W. et al. Annu. Rev. Biochem. 2009, 78:1017-1040.

44- Björkhem I. et al. Mol. Aspects Med. 2009, 30:171-179.

45- Papassotiropoulos A. et al. J. Psychiatr. Res. 2002, 36:27-32.

46- Babiker A. and Diczfalusy U. Biochim. Biophys. Acta 1998, 1392:333-339.

47- Burkard I. et al. Atherosclerosis 2007, 194:71-78.

48- Simons M. et al. Proc. Natl. Acad. Sci. U.S.A. 1998, 95:6460-6464.

49- Kölsch H. et al. Brain Res. 1999, 818:171-175.

50- Kölsch H. et al. J. Neutral Transom. 2001, 108:475-488.

51- Chauhan N.B. J. Lipid Res. 2003, 44:2019-2029.

52- Hooijmans C.R. and Kiliaan A.J. Eur. J. Pharmacol. 2008, 585:176-196.

53- Wang Y. et al. Biochim. Biophys. Acta - Mol. Cell Biol. Lipids 2010, 1801:917-923.

54- Tian G. et al. J. Neurochem. 2010, 113:978-89.

55- Fonseca A.C.R.G. et al. Exp. Neurol. 2010, 223:282-293.

56- Yamanaka K. et al. J. Biol. Chem. 2011, 286:24666-24673.

57- Schönknecht P. et al. Neurosci. Lett. 2002, 324:83-85.

58- Shafaati M. et al. Neurosci. Lett. 2007, 425:78-82.

59- Hughes T.M. et al. J. Alzheimers Dis. 2012, 30:53-61.

60- Albers J.J. et al. Int. J. Clin. Lab. Res. 1992, 22:169-72.

61- Demeester N. et al. J. Lipid Res. 2000, 41: 963-974.

62- Salvatore A. et al. J. Neurochem. 2009, 110:255-263.

63- Collet X. et al. Biochem. Biophys. Res. Commun. 1999, 258:73-76.

64- Lin Yang L. et al. Lawrence Berkeley National Laboratory, 2003, http://escholarship.org/uc/item/3p69j7f3.

65- Hirsch-Reinshagen V. et al. J. Lipid Res. 2009, 50:885-893.

66- Jonas A. Biochim. Biophys. Acta 2000, 1529:245-256.

67- Kunnen S. and Van Eck M. J. Lipid Res. 2012, 53:1783-99.

68- Cigliano L. et al. Acta Physiol. Suppl 681, 2010, 200:P49.

69- Rye K.-A. et al. J. Lipid Res. 2006, 47:1025-1036.

70- Pitas R.E. et al. Biochim. Biophys. Acta 1987, 917:148-161.

71- Zlokovic B.V. et al. Proc. Natl. Acad. Sci. USA 1996, 93:4229-4234.

72- Koch S. et al. J. Lipid Res. 2001,42:1143-1151.

73- Dietschy J.M. and Turley S.D. J. Lipid Res. 2004, 45:1375-1397.

74- Björkhem I. J. Intern. Med. 2006, 260: 493-508.

75- Elliott D.A. et al. Clin. Lipidol. 2010, 51:555-573.

76- Umeda T. et al. J. Neurosci. Res. 2010, 88:1985-94.

77- Dziegielewska K.M. et al. Dev. Biol. 1986, 115: 93-104.

78- D'Armiento J. et al. Gene 1997, 195:19 -27.

79- Sanchez D.J. et al. Neurosci. Lett. 2001, 303:181-184.

80- Lee M.Y. et al. J. Cereb. Blood Flow Metab. 2002, 22:1176 -1180.

81- Zhao X. et al. J. Neurosci. 2009, 29:15819 -15827

82- Salvatore A. et al. Biochemistry 2007, 46:11158-11168.

83- Salvatore A. et al. J. Neurochem. 2009, 110:255-263.

84- Jung S.M. et al. Neurosci. Lett. 2008, 436:153-157.

85- Tumani H. et al. Neurosci. Lett. 2009, 452:214-217.

86- Chen C. and Loo G. Lipids 1995, 30:627-631.

87- Graham A. et al. FEBS Lett. 1998, 431:327-332.

88- Subbaiah P.V. and Liu M. Biochim. Biophys. Acta 1996, 1301:115-126.

89- Bielicki J.K. et al. J. Lipid Res. 1996, 37:1012-1021.

90- Davit-Sprout A. et al. FEBS Lett. 1999, 447:106-110.

91- Wang K. and Subbaiah P.V. Biochim. Biophys. Acta 2000, 1488:268-277.

92- Hozumi I. et al. J. Neurol. Sci. 2011, 303:95-99.

93- Ohyama Y. et al. J. Biol. Chem. 2006, 281:3810-3820.

94- Cartagena C.M. et al. J Neurotraum. 2008, 25:1087-98.

95- Shafaati M. et al. Biochem. Biophys. Res. Commun. 2009, 378:689 - 694.

96- Nunes M. J. et al. J. Neurochem. 2010, 113:418 - 431.

97- Polman C. et al. Necrology 2010, 74:427-434.

98- Lauretani F. et al. Drugs and Therapy Studies 2011, 1:e6; doi: 10.4081/dts.2011.e6.

99- Szymanski P. et al. Nucl. Med. Rev. Cent. East Eur. 2010, 13:23-31.

100- Montalban X. et al. Trends Neurosci. 2011, 34:430-442.

101- Renard D. et al. Acta Neurol. Belg. 2011, 111:306-9.

102- Mosconi L. and McHugh P.F. Q. J. Nucl. Med. Mol. Imaging 2011, 55:250-64.

103- Borghammer P. Dan. Med. J. 2012, 59:B4466.

104- Blinkenberg M. et al. Neurol. Res. 2012, 34:52-8.

105- Cistaro A. et al. Eur. J. Nucl. Med. Mol. Imaging 2012, 39:251-9.

106- Hellwig S. et al. Necrology 2012, 79:1314-22.

107- Nordberg A. et al. Eur. J. Nucl. Med. Mol. Imaging 2013, 40:104-114.

108- Mirra S.S. Neurobiol. Aging 1997, 18:591-594.

109- Durand-Martel P. et al. Can. J. Neurol Sci. 2010, 37:336-42.

110- McKhann G.M. et al. Alzheimers Dement. 2011, 7:263-269.

111- Croisile B. et al. Rev. Neuron. (Paris) 2012, 168:471-482.

112- Sperling R.A. et al. Alzheimers Dement. 2011, 7:280-292.

113- De Jager P.L. et al. Lancet Neurol. 2009, 8:1111-1119.

114- Miller D.H. et al. Mult. Scler. 2008, 14:1157-1174.

115- Brex P.A. et al. N. Engl. J. Med. 2002, 346:158-64.

116- Zephir H. et al.; GRESEP (Groupe de Reflexion sur la Sclerose en Plaques; Multiple Sclerosis Think Tank). Rev. Neurol. (Paris) 2012, 168:328-37.

117- Ouallet J.C. et al. Rev. Neurol. (Paris) 2013,169 :37-46.

118- Goldstein D.S. et al. Brain 2012, 135:1900-13.

119- von Neuhoff N. et al. PLoS One 2012, 7:e44401; doi: 10.1371/journal.pone.0044401.

120- Leoni V. et al. Brain 2008, 131:2851-2859.

121- Nardo G. et al. PLoS ONE 2011, 6: e25545; doi:10.1371/journal.pone.0025545.

122- Forlenza O.V. et al. BMC Med. 2010, 8:89-102.

123- Song F. et al. Brain Res. Rev. 2009, 61:69-80.

124- Galasko D. and Montine T.J. Biomark. Med. 2010, 4:27-36.

125- Cedazo-Minguez A. and Winblad B. Exp. Gerontol. 2010, 45:5-14.

126- Cummings J.L. Alzheimers Dement. 2011, e13-e44; doi:10.1016/j.jalz.2010.06.004.

127- Ohrfelt A. et al. Dement. Geriatr. Cogn. Dis. Extra. 2011, 1:31-42.

128- Humpel C. Trends Biotechnol. 2011, 29:26-32.

129- Holtzman D.M. Neurobiol. Aging 2011, 32(Suppl 1): S4-S9.

130- Reddy M.M. et al. Cell 2011, 144:132-142.

131- Bazenet C. and Lovestone S. Biomark. Med. 2012, 6:441-454.

132- Fagan A.M. and Perrin R.J. Biomark. Med. 2012, 6:455-476.

133- Blennow K. et al. Cold Spring Harb. Perspect. Med. 2012, doi: 10.1101/cshperspect.a006221.

134- Gonzalez-Dominguez R. et al. Chem. Pap. 2012, 66:829-835.

135- Sato Y. et al. J. Lipid Res. 2012, 53:567-76.

136- Ghidoni R. et al. Clin. Biochem. 2013, 46:480-486.

137- Huang Y. and Mucke L. Cell 2012, 148:1204-1222.

138- Holtzman D.M. et al. Cold Spring Harb. Perspect. Med. 2012, doi: 10.1101/cshperspect.a006312.

139- Teunissen C.E. et al. Neurobiol. Aging 2003, 24:147-15.

140- Leoni V. et al. J. Lipid Res. 2005, 46:191-195.

141- Leoni V. et al. Neurosci. Lett. 2006, 397:83-87.

142- Björkhem I. Lipids 2007, 42:5-14.

143- Leoni V. and Caccia C. Chem. Phys. Lipids 2011, 164:515-524.

144- Hughes T.M. et al. J. Alzheimers Dis. 2012, 30:53-61.

145- Poli G. et al. Redox Biology 2013, 1:125-130.

146- El Andaloussi-Lilja J. et al. Neurochem. Int. 2009, 55:768-774.

147- Albers J.J. et al. Method Enzymol. 1986, 129:763-783.

148- Matz C.E. and Ana Jonas A. J. Biol. Chem. 1982, 257:4535-4540.

149- Hozumi I. et al. J. Neurol. Sci. 2011, 303:95-99.

150- Tallaksen C.M. et al. Am. J. Clin. Nutr. 1992, 56:559-564.

151- Reiber H. et al. Clin. Chim. Acta 1993, 217:163-173.

152- Bowman G.L. et al. J. Alzheimers Dis. 2009, 16:93-98

153- Kwon D. et al. J. Neuroimmunol. 2001, 113:1-9.

154- Geracitano R. et al. J. Physiol. 2005, 568.1:97-110.

155- Chang S. et al. Neurosci. Lett. 2008, 441:134-138.

156- Nisticò R. et al. Br. J. Pharmacol. 2008, 153:1022-1029.

157- Kurtzke JF. Neurology 1983, 33:1444-52.

158- McKhann G. et al. Neurology 1984, 34:939-944.

159- Brooks B.R. et al. Amyotroph. Lateral Scler. Other Motor Neuron Disord. 2000, 1:293-299.

160- Blennow K. et al. Eur. Neurol. 1993, 33:129-33.

161- Burkard I. et al. J. Lipid Res. 2004, 45:776-781.

162- Cigliano L. et al. FEBS J. 2009, 276:6158-6171.

163- Rye K.A. et al. J. Lipid Res. 2006, 47:1025-1036.

164- Porta A. et al. Zygote 1999, 7:67-77.

165- Carlucci A. et al. J. Physiol. Biochem. 2012, 68:541-53.

166- Yamauchi K. et al. Clin. Chem. 1999, 45:1431-1438.

167- Riddell D.R. et al. J. Neurosci. 2008, 28:11445-11453.

168- Elliott D.A. et al. BMC Neurosci. 2010, 11:23-32.

**Claims**

1. A method for the diagnosis and/or prognosis of a neurodegenerative disease and/or for the monitoring of the efficacy of a therapeutic treatment of a neurodegenerative disease and/or for the screening of a therapeutic treatment of a neurodegenerative disease comprising the step of measuring the amount of esterified cerebrosterol [24(S)OH-CE] in an isolated biological sample from a subject and comparing the same with a proper value from a control sample.

2. The method according to claim 1 comprising the steps of:

   a) measuring the amounts of total cerebrosterol [24(S)OH-$C_{TOT}$] and of non-esterified cerebrosterol [24(S)OH-$C_{NE}$];

b) calculating the amount of [24(S)OH-CE] according to the following formula:

$$[24(S)OH-CE] = [24(S)OH-C_{TOT}] - [24(S)OH-C_{NE}];$$

c) comparing the obtained value as in step b) with the proper value obtained from a control sample.

3. The method according to claim 1 or 2 comprising the steps of:

a) introducing a predetermined amount of deuterated $24(S)OH-C_{NE}$, as an internal standard, into the isolated biological sample;
b) separating said sample into a first and a second aliquot;
c) treating said first aliquot by means of saponification;
d) optionally, extracting and isolating $24(S)OH-C_{NE}$ from each of said first and second aliquot;
e) measuring the amount of $24(S)OH-C_{NE}$ in said first and said second aliquot and standardising obtained values with the measurement of the amounts of the internal standard, wherein the amount of $24(S)OH-C_{NE}$ in the first aliquot corresponds to $[24(S)OH-C_{TOT}]$ and the amount of $24(S)OH-C_{NE}$ in the second aliquot corresponds to $[24(S)OH-C_{NE}]$;
f) calculating the amount of [24(S)OH-CE] according to the formula $[24(S)OH-CE] = [24(S)OH-C_{TOT}] - [24(S)OH-C_{NE}]$;
g) optionally calculating the ratio $[24(S)OH-CE]/[24(S)OH-C_{NE}]$ and/or $[24(S)OH-CE]/[24(S)OH-C_{TOT}]$, and/or the percentage $[24(S)OH-CE] \times 100/[24(S)OH-C_{TOT}]$;
h) comparing values as obtained in f) or g) with proper values obtained from a control sample.

4. The method according to any of previous claims, wherein the amount of [24(S)OH-CE] is related to the activity of the lecithin-cholesterol acyltransferase (LCAT) in the central nervous system.

5. The method according to any of previous claims, wherein the neurodegenerative disease is selected from the group consisting of: Alzheimer's disease, multiple sclerosis, amyotrophic lateral sclerosis, Parkinson's disease or Huntington's disease.

6. The method according to any one of the previous claims, wherein the biological sample is selected from the group consisting of: serum, plasma, CSF, saliva, urine or fluid of the hair bulb.

7. The method according to any one of the previous claims, wherein the amount of $24(S)OH-C_{NE}$ is measured by means of specific antibody or coulometric or electrochemical detector.

8. Use of the biomarker esterified cerebrosterol [24(S)OH-CE] in an in vitro method for the diagnosis and/or prognosis of a neurodegenerative disease and/or for the monitoring of the efficacy of a therapeutic treatment of a neurodegenerative disease and/or for the screening of a therapeutic treatment of a neurodegenerative disease.

9. Use of the biomarker esterified cerebrosterol [24(S)OH-CE] in an in vitro method for the diagnosis and/or prognosis of a neurodegenerative disease and/or for the monitoring of the efficacy of a therapeutic treatment of a neurodegenerative disease and/or for the screening of a therapeutic treatment of a neurodegenerative disease, said method being defined in claims 1 to 7.

10. Use of esterified cerebrosterol [24(S)OH-CE] to determined, in vitro, the ratios $[24(S)OH-CE]/[24(S)OH-C_{NE}]$ or $[24(S)OH-CE]/[24(S)OH-C_{TOT}]$ or of the percentage $[24(S)OH-CE] \times 100/[24(S)OH-C_{TOT}]$ for the diagnosis and/or prognosis of a neurodegenerative disease and/or for the monitoring of the efficacy of a therapeutic treatment of a neurodegenerative disease and/or for the screening of a therapeutic treatment of a neurodegenerative disease and/or for measurement of the activity of lecithin-cholesterol acyltransferase (LCAT).

11. The use according to claims 8-10, wherein the neurodegenerative disease is selected from the group consisting of: Alzheimer's disease, multiple sclerosis, amyotrophic lateral sclerosis, Parkinson's disease and Huntington's disease.

12. Use of a kit comprising:

- means to measure the amount of at least one biomarker as defined in claim 8 or 9 and optionally

- control means

for working the method of any one of claims 1-7.

**Patentansprüche**

1. Verfahren zur Diagnose und/oder Prognose einer neurodegenerativen Erkrankung und/oder zur Überwachung der Wirksamkeit einer therapeutischen Behandlung einer neurodegenerativen Erkrankung und/oder für das Screening einer therapeutischen Behandlung einer neurodegenerativen Erkrankung, die den Schritt des Messens der Menge an verestertem Cerebrosterol [24(S)OH-CE] in einer isolierten biologischen Probe von einem Subjekt umfasst und das Vergleichen davon mit einem geeigneten Wert einer Kontrollprobe.

2. Verfahren nach Anspruch 1, das die folgenden Schritte umfasst:

   a) Messen der Mengen an Gesamt-Cerebrosterol [24(S)OH-C$_{TOT}$] und an nicht-verestertem Cerebrosterol [24(S)OH-C$_{NE}$];
   b) Berechnen der Menge von [24(S)OH-CE] nach der folgenden Formel:

$$[24(S)OH-CE] = [24(S)OH-C_{TOT}] - [24(S)OH-C_{NE}];$$

   c) Vergleichen des in Schritt b) erhaltenen Wertes mit dem geeigneten Wert, der von einer Kontrollprobe erhalten wurde.

3. Verfahren nach Anspruch 1 oder 2, das die folgenden Schritte umfasst:

   a) Einführen einer vorbestimmten Menge an deuteriertem 24(S)OH-C$_{NE}$, als interner Standard, in die isolierte biologische Probe;
   b) Auftrennen der Probe in ein erstes und ein zweites Aliquot;
   c) Behandeln des ersten Aliquots mittels Verseifung;
   d) wahlweise Extrahieren und Isolieren von 24(S)OH-C$_{NE}$ aus sowohl dem ersten als auch dem zweiten Aliquot;
   e) Messen der Menge an 24(S)OH-C$_{NE}$ in dem ersten und dem zweiten Aliquot und Standardisieren der erhaltenen Werte mit der Messung der Mengen des internen Standards, wobei die Menge an 24(S)OH-C$_{NE}$ im ersten Aliquot [24(S)OH-C$_{TOT}$] entspricht und die Menge an 24(S)OH-C$_{NE}$ im zweiten Aliquot [24(S)OH-C$_{NE}$] entspricht;
   f) Berechnen der Menge an [24(S)OH-CE] nach der Formel [24(S)OH-CE] = [24(S)OH-C$_{TOT}$] - [24(S)OH-C$_{NE}$];
   g) wahlweise Berechnen des Verhältnisses [24(S)OH-CE] / [24(S)OH-C$_{NE}$] und/oder [24(S)OH-CE] / [24(S)OH-C$_{TOT}$] und/oder des Prozentsatzes [24(S)OH-CE] x 100 / [24(S)OH-C$_{TOT}$];
   h) Vergleichen der in f) oder g) erhaltenen Werte mit geeigneten Werten, die von einer Kontrollprobe erhalten wurden.

4. Verfahren nach einem der vorherigen Ansprüche, wobei die Menge an [24(S)OH-CE] in Zusammenhang steht mit der Aktivität der Lecithin-Cholesterin Acyltransferase (LCAT) im zentralen Nervensystem.

5. Verfahren nach einem der vorherigen Ansprüche, wobei die neurodegenerative Erkrankung ausgewählt ist aus der Gruppe, bestehend aus: Alzheimer Krankheit, Multipler Sklerose, amyotropher Lateralsklerose, Parkinson Krankheit oder Huntington Krankheit.

6. Verfahren nach einem der vorherigen Ansprüche, wobei die biologische Probe ausgewählt ist aus der Gruppe, bestehend aus: Serum, Plasma, Liquor, Speichel, Urin oder Flüssigkeit der Haarzwiebel.

7. Verfahren nach einem der vorherigen Ansprüche, wobei die Menge an 24(S)OH-C$_{NE}$ mittels spezifischer Antikörper oder eines kolometrischen oder elektrochemischen Detektors gemessen wird.

8. Verwendung des Biomarkers verestertes Cerebrosterol [24(S) OH-CE] in einem in-vitro-Verfahren zur Diagnose und/oder Prognose einer neurodegenerativen Erkrankung und/oder zur Überwachung der Wirksamkeit einer therapeutischen Behandlung einer neurodegenerativen Erkrankungund/oder für das Screening einer therapeutischen Behandlung einer neurodegenerativen Erkrankung.

9. Verwendung des Biomarkers verestertes Cerebrosterol [24(S)OH-CE] in einem in-vitro-Verfahren zur Diagnose und/oder Prognose einer neurodegenerativen Erkrankung und/oder zur Überwachung der Wirksamkeit einer therapeutischen Behandlung einer neurodegenerativen Erkrankungund/oder für das Screening einer therapeutischen Behandlung einer neurodegenerativen Erkrankung, wobei das Verfahren in den Ansprüchen 1 bis 7 definiert ist.

10. Verwendung von verestertem Cerebrosterol [24(S)OH-CE], um in vitro
die Verhältnisse [24(S)OH-CE] / [24(S)OH-C$_{NE}$] oder [24(S)OH-CE] / [24(S)OH-C$_{TOT}$] oder den Prozentsatz [24(S)OH-CE] x 100 / [24 (S) OH-C$_{TOT}$] zu bestimmen zur Diagnose und/oder Prognose einer neurodegenerativen Erkrankung und/oder zur Überwachung der Wirksamkeit einer therapeutischen Behandlung einer neurodegenerativen Erkrankung und/oder zum Screening einer therapeutischen Behandlung einer neurodegenerativen Erkrankung und/oder zur Messung der Aktivität von Lecithin-Cholesterin Acyltransferase (LCAT).

11. Verwendung nach den Ansprüchen 8-10, wobei die neurodegenerative Erkrankung ausgewählt ist aus der Gruppe, bestehend aus: Alzheimer Krankheit, Multipler Sklerose, amyotropher Lateralsklerose, Parkinson Krankheit und Huntington Krankheit.

12. Verwendung eines Kits, der Folgendes umfasst:

- Mittel zum Messen der Menge von mindestens einem Biomarker wie in Anspruch 8 oder 9 definiert und wahlweise
- Kontrollmittel

zur Durchführung des Verfahrens nach einem der Ansprüche 1-7.

**Revendications**

1. Procédé de diagnostic et/ou de pronostic d'une maladie neurodégénérative et/ou de surveillance de l'efficacité d'un traitement thérapeutique d'une maladie neurodégénérative et/ou de criblage d'un traitement thérapeutique d'une maladie neurodégénérative comprenant l'étape de mesure de la quantité de cérébrostérol estérifié [24(S)OH-CE] dans un échantillon biologique isolé d'un sujet et de comparaison de celle-ci à une valeur correcte d'un échantillon témoin.

2. Procédé selon la revendication 1, comprenant les étapes de :

a) mesure des quantités de cérébrostérol total [24(S)OH-C$_{TOT}$] et de cérébrostérol non estérifié [24(S)OH-C$_{NE}$] ;
b) calcul de la quantité de [24(S)OH-CE] selon la formule suivante :

$$[24(S)OH-CE] = [24(S)OH-C_{TOT}] - [24(S)OH-C_{NE}] ;$$

c) comparaison de la valeur obtenue comme à l'étape b) à la valeur correcte obtenue d'un échantillon témoin.

3. Procédé selon la revendication 1 ou 2, comprenant les étapes de :

a) introduction d'une quantité prédéterminée de 24(S)OH-C$_{NE}$ deutéré, en tant qu'étalon interne, dans l'échantillon biologique isolé ;
b) séparation dudit échantillon en une première et une seconde aliquote ;
c) traitement de ladite première aliquote par saponification ;
d) facultativement, extraction et isolement de 24(S)OH-C$_{NE}$ de chacune desdites première et seconde aliquotes ;
e) mesure de la quantité de 24(S)OH-C$_{NE}$ dans ladite première et ladite seconde aliquote et standardisation des valeurs obtenues avec la mesure des quantités de l'étalon interne, dans lequel la quantité de 24(S)OH-C$_{NE}$ dans la première aliquote correspond au [24(S)OH-C$_{TOT}$] et la quantité de 24(S)OH-C$_{NE}$ dans la seconde aliquote correspond au [24(S)OH-C$_{NE}$] ;
f) calcul de la quantité de [24(S)OH-CE] selon la formule [24(S)OH-CE] = [24(S)OH-C$_{TOT}$] - [24(S)OH-C$_{NE}$] ;
g) facultativement calcul du rapport [24(S)OH-CE]/[24(S)OH-C$_{NE}$] et/ou [24(S)OH-CE]/[24(S)OH-C$_{TOT}$], et/ou du pourcentage [24(S)OH-CE] x 100/[24(S)OH-C$_{TOT}$] ;
h) comparaison des valeurs telles qu'obtenues à l'étape f) ou g) à des valeurs correctes obtenues d'un échantillon témoin.

4. Procédé selon l'une quelconque des revendications précédentes, dans lequel la quantité de [24(S)OH-CE] est liée à l'activité de la lécithine-cholestérol acyltransférase (LCAT) dans le système nerveux central.

5. Procédé selon l'une quelconque des revendications précédentes, dans lequel la maladie neurodégénérative est sélectionnée dans le groupe constitué de : la maladie d'Alzheimer, la sclérose en plaques, la sclérose latérale amyotrophique, la maladie de Parkinson ou la maladie d'Huntington.

6. Procédé selon l'une quelconque des revendications précédentes, dans lequel l'échantillon biologique est sélectionné dans le groupe constitué : du sérum, du plasma, du LCR, de la salive, de l'urine ou du fluide des bulbes capillaires.

7. Procédé selon l'une quelconque des revendications précédentes, dans lequel la quantité de $24(S)OH-C_{NE}$ est mesurée au moyen d'un anticorps spécifique ou d'un détecteur coulométrique ou d'un détecteur électrochimique.

8. Utilisation du biomarqueur cérébrostérol estérifié [24(S)OH-CE] dans un procédé in vitro de diagnostic et/ou de pronostic d'une maladie neurodégénérative et/ou de surveillance de l'efficacité d'un traitement thérapeutique d'une maladie neurodégénérative et/ou de criblage d'un traitement thérapeutique d'une maladie neurodégénérative.

9. Utilisation du biomarqueur cérébrostérol estérifié [24(S)OH-CE] dans un procédé *in vitro* de diagnostic et/ou de pronostic d'une maladie neurodégénérative et/ou de surveillance de l'efficacité d'un traitement thérapeutique d'une maladie neurodégénérative et/ou de criblage d'un traitement thérapeutique d'une maladie neurodégénérative, ledit procédé étant défini dans les revendications 1 à 7.

10. Utilisation de cérébrostérol estérifié [24(S)OH-CE] pour déterminer, *in vitro,* les rapports de [24(S)OH-CE]/[24(S)OH-$C_{NE}$] ou [24(S)OH-CE]/[24(S)OH-$C_{TOT}$] ou du pourcentage [24(S)OH-CE] x 100/[24(S)OH-$C_{TOT}$] pour le diagnostic et/ou le pronostic d'une maladie neurodégénérative et/ou pour la surveillance de l'efficacité d'un traitement thérapeutique d'une maladie neurodégénérative et/ou pour le criblage d'un traitement thérapeutique d'une maladie neurodégénérative et/ou pour la mesure de l'activité de la lécithine-cholestérol acyltransférase (LCAT).

11. Utilisation selon les revendications 8 à 10, dans laquelle la maladie neurodégénérative est sélectionnée dans le groupe constitué de : la maladie d'Alzheimer, la sclérose en plaques, la sclérose latérale amyotrophique, la maladie de Parkinson et la maladie d'Huntington.

12. Utilisation d'un kit, comprenant :

- un moyen de mesure de la quantité d'au moins un biomarqueur tel que défini dans la revendication 8 ou 9 et facultativement
- un moyen de commande,

pour exécuter le procédé selon l'une quelconque des revendications 1 à 7.

Fig. 1

Fig. 2

Fig. 3

A : cells incubated in culture medium alone (control)
B : cells treated with LCAT and ApoA-I/PC in culture medium
C : cells treated with 24(S)OH-C$_{NE}$ in culture medium
D : cells treated with 24(S)OH-C$_{NE}$, LCAT and ApoA-I/PC in culture medium

Fig. 4

Fig. 5

Fig. 6

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012064501 A
- US 2009286272 E
- US 2009286272 A

**Non-patent literature cited in the description**

- **ISCHIROPOULOS H. ; BECKMAN J.S.** *J. Clin. Invest.,* 2003, vol. 111, 163-169
- **BJÖRKHEM I. ; MEANEY S.** *Arterioscler. Thromb. Vasc. Biol.,* 2004, vol. 24, 806-815
- **HALLIWELL B.** *J. Neurochem.,* 2006, vol. 97, 1634-1658
- **OKOUCHI M. et al.** *Antioxid. Redox Sign.,* 2007, vol. 9, 1059-1096
- **ADIBHATLA R.A. ; HATCHER J.F.** *Subcell. Biochem.,* 2008, vol. 49, 241-68
- **DIETSCHY J.M.** *Biol. Chem.,* 2009, vol. 390, 287-293
- **AMOR S. et al.** *Immunology,* 2010, vol. 129, 154-169
- **GLASS C.K. et al.** *Cell,* 2010, vol. 140, 918-934
- **LIU J.-P. et al.** *Mol. Cell. Neurosci.,* 2010, vol. 43, 33-42
- **SHUKLA V. et al.** *Adv. Pharmacol. Sci.,* 2011, vol. 2011, 1-13
- **FREARS E.R. et al.** *Neuroreport,* 1999, vol. 10, 1699-1705
- **KOUDINOV A.R. ; KOUDINOVA N.V.** *Clin. Med. Health Res.,* 2001, http://clinmed.netprints.org/cgi/content/full/2001100005v1
- **CHAUHAN N.B.** *J. Lipid Res.,* 2003, vol. 44, 2019-2029
- **PUGLIELLI L. et al.** *Nat. Neurosci.,* 2003, vol. 6, 345-351
- **CASSERLY I. ; TOPOL E.** *Lancet,* 2004, vol. 363, 1139-46
- **CUTLER R.G. et al.** *Proc. Natl. Acad. Sci. USA,* 2004, vol. 101, 2070-2075
- **CARTER C.J.** *Neurochem. Int.,* 2007, vol. 50, 12-38
- **HIRSCH-REINSHAGEN V. ; WELLINGTON C.L.** *Curr. Opin. Lipidol.,* 2007, vol. 18, 325-332
- **XIONG H. et al.** *Neurobiol. Dis.,* 2008, vol. 29, 422-437
- Cholesterol in Alzheimer's Disease and other Amyloidogenic Disorders. In: ''Cholesterol Binding and Cholesterol Transport Proteins. Structure and Function in Health and Disease. **HARRIS J.R. ; MILTON N.G.N.** Subcellular Biochemistry. Springer Verlag, 2010, vol. 51, 47-75
- **MATHEW A. et al.** *Brain Res. Bull.,* 2011, vol. 86, 1-12
- **RICCIARELLI R. et al.** *IUBMB Life,* 2012, vol. 64, 931-935
- **GAMBA P. et al.** *Ann. N.Y. Acad. Sci.,* 2012, vol. 1259, 54-64
- **PAPPOLLA M.A. et al.** *Free Radic. Biol. Med.,* 2002, vol. 33, 173-181
- **NELSON T.J. ; ALKON D.L.** *J. Biol. Chem.,* 2005, vol. 280, 7377-7387
- **GAMBA P. et al.** *Aging Cell,* 2011, vol. 10, 403-417
- **IANNILLI F. et al.** *Neurobiol. Aging,* 2011, vol. 32, 1033-1042
- **SODERO A.O. et al.** *Neurobiol. Aging,* 2011, vol. 32, 1043-1053
- **WANG Y. et al.** *J. Proteome Res.,* 2008, vol. 7, 1606-1614
- **BROWN A.J. ; JESSUP W.** *Mol. Aspects Med.,* 2009, vol. 30, 111-122
- **HIRSCH-REINSHAGEN V. et al.** *Mol. Cell. Biochem.,* 2009, vol. 326, 121-129
- **NIEWEG K. et al.** *J. Neurochem.,* 2009, vol. 109, 125-134
- **UMEDA T. et al.** *J. Neurosci. Res.,* 2010, vol. 88, 1985-1994
- **CARTAGENA C.M. et al.** *Brain Res.,* 2010, vol. 1319, 1-12
- **PFRIEGER F.W. ; UNGERER N.** *Progr. Lipid Res.,* 2011, vol. 50, 357-371
- **LEONI V. ; CACCIA C.** *Biochimie,* 2013, vol. 95, 595-612
- **PITAS R.E. et al.** *J. Biol. Chem.,* 1987, vol. 262, 14352-14360
- **DZELETOVIC S. et al.** *Anal. Biochem.,* 1995, vol. 225, 73-80

- **DEMEESTER N. et al.** *J. Lipid Res.,* 2000, vol. 41, 963-974
- **XIE C. et al.** *J. Lipid Res.,* 2003, vol. 44, 1780-1789
- **LÜTJOHANN D. et al.** *Proc. Natl. Acad. Sci. USA,* 1996, vol. 93, 9799-9804
- **BJÖRKHEM I.** *J. Intern. Med.,* 2006, vol. 260, 493-508
- **RUSSELL D.W. et al.** *Annu. Rev. Biochem.,* 2009, vol. 78, 1017-1040
- **BJÖRKHEM I. et al.** *Mol. Aspects Med.,* 2009, vol. 30, 171-179
- **PAPASSOTIROPOULOS A. et al.** *J. Psychiatr. Res.,* 2002, vol. 36, 27-32
- **BABIKER A. ; DICZFALUSY U.** *Biochim. Biophys. Acta,* 1998, vol. 1392, 333-339
- **BURKARD I. et al.** *Atherosclerosis,* 2007, vol. 194, 71-78
- **SIMONS M. et al.** *Proc. Natl. Acad. Sci. U.S.A.,* 1998, vol. 95, 6460-6464
- **KÖLSCH H. et al.** *Brain Res.,* 1999, vol. 818, 171-175
- **KÖLSCH H. et al.** *J. Neutral Transom.,* 2001, vol. 108, 475-488
- **HOOIJMANS C.R. ; KILIAAN A.J.** *Eur. J. Pharmacol.,* 2008, vol. 585, 176-196
- **WANG Y. et al.** *Biochim. Biophys. Acta - Mol. Cell Biol. Lipids,* 2010, vol. 1801, 917-923
- **TIAN G. et al.** *J. Neurochem.,* 2010, vol. 113, 978-89
- **FONSECA A.C.R.G. et al.** *Exp. Neurol.,* 2010, vol. 223, 282-293
- **YAMANAKA K. et al.** *J. Biol. Chem.,* 2011, vol. 286, 24666-24673
- **SCHÖNKNECHT P. et al.** *Neurosci. Lett.,* 2002, vol. 324, 83-85
- **SHAFAATI M. et al.** *Neurosci. Lett.,* 2007, vol. 425, 78-82
- **HUGHES T.M. et al.** *J. Alzheimers Dis.,* 2012, vol. 30, 53-61
- **ALBERS J.J. et al.** *Int. J. Clin. Lab. Res.,* 1992, vol. 22, 169-72
- **SALVATORE A. et al.** *J. Neurochem.,* 2009, vol. 110, 255-263
- **COLLET X. et al.** *Biochem. Biophys. Res. Commun.,* 1999, vol. 258, 73-76
- **LIN YANG L. et al.** *Lawrence Berkeley National Laboratory,* 2003, http://escholarship.org/uc/item/3p69j7f3
- **HIRSCH-REINSHAGEN V. et al.** *J. Lipid Res.,* 2009, vol. 50, 885-893
- **JONAS A.** *Biochim. Biophys. Acta,* 2000, vol. 1529, 245-256
- **KUNNEN S. ; VAN ECK M.** *J. Lipid Res.,* 2012, vol. 53, 1783-99
- **CIGLIANO L. et al.** *Acta Physiol. Suppl,* 2010, vol. 681 (200), 49
- **RYE K.-A. et al.** *J. Lipid Res.,* 2006, vol. 47, 1025-1036
- **PITAS R.E. et al.** *Biochim. Biophys. Acta,* 1987, vol. 917, 148-161
- **ZLOKOVIC B.V. et al.** *Proc. Natl. Acad. Sci. USA,* 1996, vol. 93, 4229-4234
- **KOCH S. et al.** *J. Lipid Res.,* 2001, vol. 42, 1143-1151
- **DIETSCHY J.M. ; TURLEY S.D.** *J. Lipid Res.,* 2004, vol. 45, 1375-1397
- **ELLIOTT D.A. et al.** *Clin. Lipidol.,* 2010, vol. 51, 555-573
- **UMEDA T. et al.** *J. Neurosci. Res.,* 2010, vol. 88, 1985-94
- **DZIEGIELEWSKA K.M. et al.** *Dev. Biol.,* 1986, vol. 115, 93-104
- **D'ARMIENTO J. et al.** *Gene,* 1997, vol. 195, 19-27
- **SANCHEZ D.J. et al.** *Neurosci. Lett.,* 2001, vol. 303, 181-184
- **LEE M.Y. et al.** *J. Cereb. Blood Flow Metab.,* 2002, vol. 22, 1176-1180
- **ZHAO X. et al.** *J. Neurosci.,* 2009, vol. 29, 15819-15827
- **SALVATORE A. et al.** *Biochemistry,* 2007, vol. 46, 11158-11168
- **JUNG S.M. et al.** *Neurosci. Lett.,* 2008, vol. 436, 153-157
- **TUMANI H. et al.** *Neurosci. Lett.,* 2009, vol. 452, 214-217
- **CHEN C. ; LOO G.** *Lipids,* 1995, vol. 30, 627-631
- **GRAHAM A. et al.** *FEBS Lett.,* 1998, vol. 431, 327-332
- **SUBBAIAH P.V. ; LIU M.** *Biochim. Biophys. Acta,* 1996, vol. 1301, 115-126
- **BIELICKI J.K. et al.** *J. Lipid Res.,* 1996, vol. 37, 1012-1021
- **DAVIT-SPROUT A. et al.** *FEBS Lett.,* 1999, vol. 447, 106-110
- **WANG K. ; SUBBAIAH P.V.** *Biochim. Biophys. Acta,* 2000, vol. 1488, 268-277
- **HOZUMI I. et al.** *J. Neurol. Sci.,* 2011, vol. 303, 95-99
- **OHYAMA Y. et al.** *J. Biol. Chem.,* 2006, vol. 281, 3810-3820
- **CARTAGENA C.M. et al.** *J Neurotraum.,* 2008, vol. 25, 1087-98
- **SHAFAATI M. et al.** *Biochem. Biophys. Res. Commun.,* 2009, vol. 378, 689-694
- **NUNES M. J. et al.** *J. Neurochem.,* 2010, vol. 113, 418-431
- **POLMAN C. et al.** *Necrology,* 2010, vol. 74, 427-434
- **LAURETANI F. et al.** *Drugs and Therapy Studies,* 2011, vol. 1, e6
- **SZYMANSKI P et al.** *Nucl. Med. Rev. Cent. East Eur.,* 2010, vol. 13, 23-31

- **MONTALBAN X. et al.** *Trends Neurosci.,* 2011, vol. 34, 430-442
- **RENARD D. et al.** *Acta Neurol. Belg.,* 2011, vol. 111, 306-9
- **MOSCONI L. ; MCHUGH P.F. Q.** *J. Nucl. Med. Mol. Imaging,* 2011, vol. 55, 250-64
- **BORGHAMMER P.** *Dan. Med. J.,* 2012, vol. 59, B4466
- **BLINKENBERG M. et al.** *Neurol. Res.,* 2012, vol. 34, 52-8
- **CISTARO A. et al.** *Eur. J. Nucl. Med. Mol. Imaging,* 2012, vol. 39, 251-9
- **HELLWIG S. et al.** *Necrology,* 2012, vol. 79, 1314-22
- **NORDBERG A. et al.** *Eur. J. Nucl. Med. Mol. Imaging,* 2013, vol. 40, 104-114
- **MIRRA S.S.** *Neurobiol. Aging,* 1997, vol. 18, 591-594
- **DURAND-MARTEL P. et al.** *Can. J. Neurol Sci.,* 2010, vol. 37, 336-42
- **MCKHANN G.M. et al.** *Alzheimers Dement.,* 2011, vol. 7, 263-269
- **CROISILE B. et al.** *Rev. Neuron. (Paris),* 2012, vol. 168, 471-482
- **SPERLING R.A. et al.** *Alzheimers Dement.,* 2011, vol. 7, 280-292
- **DE JAGER P.L. et al.** *Lancet Neurol.,* 2009, vol. 8, 1111-1119
- **MILLER D.H. et al.** *Mult. Scler.,* 2008, vol. 14, 1157-1174
- **BREX P.A. et al.** *N. Engl. J. Med.,* 2002, vol. 346, 158-64
- **ZEPHIR H. et al.** GRESEP (Groupe de Reflexion sur la Sclerose en Plaques; Multiple Sclerosis Think Tank). *Rev. Neurol. (Paris),* 2012, vol. 168, 328-37
- **OUALLET J.C. et al.** *Rev. Neurol. (Paris),* 2013, vol. 169, 37-46
- **GOLDSTEIN D.S. et al.** *Brain,* 2012, vol. 135, 1900-13
- **NEUHOFF N. et al.** *PLoS One,* 2012, vol. 7, e44401
- **LEONI V. et al.** *Brain,* 2008, vol. 131, 2851-2859
- **NARDO G. et al.** *PLoS ONE,* 2011, vol. 6, e25545
- **FORLENZA O.V. et al.** *BMC Med.,* 2010, vol. 8, 89-102
- **SONG F. et al.** *Brain Res. Rev.,* 2009, vol. 61, 69-80
- **GALASKO D. ; MONTINE T.J.** *Biomark. Med.,* 2010, vol. 4, 27-36
- **CEDAZO-MINGUEZ A. ; WINBLAD B.** *Exp. Gerontol.,* 2010, vol. 45, 5-14
- **CUMMINGS J.L.** *Alzheimers Dement.,* 2011, e13-e44
- **OHRFELT A. et al.** *Dement. Geriatr. Cogn. Dis. Extra.,* 2011, vol. 1, 31-42
- **HUMPEL C.** *Trends Biotechnol.,* 2011, vol. 29, 26-32
- **HOLTZMAN D.M.** *Neurobiol. Aging,* 2011, vol. 32 (1), S4-S9
- **REDDY M.M. et al.** *Cell,* 2011, vol. 144, 132-142
- **BAZENET C. ; LOVESTONE S.** *Biomark. Med.,* 2012, vol. 6, 441-454
- **FAGAN A.M. ; PERRIN R.J.** *Biomark. Med.,* 2012, vol. 6, 455-476
- **BLENNOW K. et al.** *Cold Spring Harb. Perspect. Med.,* 2012
- **GONZALEZ-DOMINGUEZ R. et al.** *Chem. Pap.,* 2012, vol. 66, 829-835
- **SATO Y. et al.** *J. Lipid Res.,* 2012, vol. 53, 567-76
- **GHIDONI R. et al.** *Clin. Biochem.,* 2013, vol. 46, 480-486
- **HUANG Y. ; MUCKE L.** *Cell,* 2012, vol. 148, 1204-1222
- **HOLTZMAN D.M. et al.** *Cold Spring Harb. Perspect. Med.,* 2012
- **TEUNISSEN C.E. et al.** *Neurobiol. Aging,* 2003, vol. 24, 147-15
- **LEONI V. et al.** *J. Lipid Res.,* 2005, vol. 46, 191-195
- **LEONI V. et al.** *Neurosci. Lett.,* 2006, vol. 397, 83-87
- **BJÖRKHEM I.** *Lipids,* 2007, vol. 42, 5-14
- **LEONI V. ; CACCIA C.** *Chem. Phys. Lipids,* 2011, vol. 164, 515-524
- **POLI G. et al.** *Redox Biology,* 2013, vol. 1, 125-130
- **EL ANDALOUSSI-LILJA J. et al.** *Neurochem. Int.,* 2009, vol. 55, 768-774
- **ALBERS J.J. et al.** *Method Enzymol.,* 1986, vol. 129, 763-783
- **MATZ C.E. ; ANA JONAS A.** *J. Biol. Chem.,* 1982, vol. 257, 4535-4540
- **TALLAKSEN C.M. et al.** *Am. J. Clin. Nutr.,* 1992, vol. 56, 559-564
- **REIBER H. et al.** *Clin. Chim. Acta,* 1993, vol. 217, 163-173
- **BOWMAN G.L. et al.** *J. Alzheimers Dis.,* 2009, vol. 16, 93-98
- **KWON D. et al.** *J. Neuroimmunol.,* 2001, vol. 113, 1-9
- **GERACITANO R. et al.** *J. Physiol.,* 2005, vol. 568 (1), 97-110
- **CHANG S. et al.** *Neurosci. Lett.,* 2008, vol. 441, 134-138
- **NISTICÒ R. et al.** *Br. J. Pharmacol.,* 2008, vol. 153, 1022-1029
- **KURTZKE JF.** *Neurology,* 1983, vol. 33, 1444-52
- **MCKHANN G. et al.** *Neurology,* 1984, vol. 34, 939-944

- **BROOKS B.R. et al.** *Amyotroph. Lateral Scler. Other Motor Neuron Disord.,* 2000, vol. 1, 293-299
- **BLENNOW K. et al.** *Eur. Neurol.,* 1993, vol. 33, 129-33
- **BURKARD I. et al.** *J. Lipid Res.,* 2004, vol. 45, 776-781
- **CIGLIANO L. et al.** *FEBS J.,* 2009, vol. 276, 6158-6171
- **RYE K.A. et al.** *J. Lipid Res.,* 2006, vol. 47, 1025-1036
- **PORTA A. et al.** *Zygote,* 1999, vol. 7, 67-77
- **CARLUCCI A. et al.** *J. Physiol. Biochem.,* 2012, vol. 68, 541-53
- **YAMAUCHI K. et al.** *Clin. Chem.,* 1999, vol. 45, 1431-1438
- **RIDDELL D.R. et al.** *J. Neurosci.,* 2008, vol. 28, 11445-11453
- **ELLIOTT D.A. et al.** *BMC Neurosci.,* 2010, vol. 11, 23-32